(19) Europäisches Patentamt / European Patent Office / Office européen des brevets

(11) **EP 4 357 362 A1**

(12) **EUROPEAN PATENT APPLICATION**
published in accordance with Art. 153(4) EPC

(43) Date of publication:
**24.04.2024 Bulletin 2024/17**

(21) Application number: **22824340.8**

(22) Date of filing: **17.06.2022**

(51) International Patent Classification (IPC):
**C07K 16/28** (2006.01)    **A61K 39/395** (2006.01)
**A61P 35/00** (2006.01)    **C12N 5/0783** (2010.01)
**C12N 15/09** (2006.01)

(52) Cooperative Patent Classification (CPC):
**A61K 39/395; A61P 35/00; C07K 16/28;
C12N 5/06; C12N 15/09**

(86) International application number:
**PCT/CN2022/099607**

(87) International publication number:
**WO 2022/262868 (22.12.2022 Gazette 2022/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.06.2021   CN 202110678326
06.06.2022   CN 202210634042**

(71) Applicant: **Harbour Biomed (Shanghai) Co., Ltd
Shanghai 201203 (CN)**

(72) Inventors:
• **WU, Xiaodong**
  **Shanghai 201203 (CN)**
• **ZHONG, Chen**
  **Shanghai 201203 (CN)**

• **DU, Fangfang**
  **Shanghai 201203 (CN)**
• **JIA, Gezi**
  **Shanghai 201203 (CN)**
• **WANG, Yongqiang**
  **Shanghai 201203 (CN)**
• **SHI, Lei**
  **Shanghai 201203 (CN)**
• **HE, Yun**
  **Shanghai 201203 (CN)**
• **RONG, Yiping**
  **Shanghai 201203 (CN)**

(74) Representative: **Murgitroyd & Company
165-169 Scotland Street
Glasgow G5 8PL (GB)**

Remarks:
The complete document including Reference
Table(s) and the Sequence Listing(s) can be
downloaded from the EPO website

(54) **BISPECIFIC ANTIBODY COMBINATION AND USE THEREOF**

(57)    Provided are a bispecific antibody combination and a use thereof. The bispecific antibody combination comprises a bispecific antibody I and a bispecific antibody II. The bispecific antibody I comprises a CD3-targeting domain and a tumor-associated antigen-targeting domain, and the bispecific antibody II comprises a 4-1BB-targeting domain and a tumor-associated antigen-targeting domain. Further provided are a pharmaceutical composition, a test kit, a kit of parts and an administration device containing the same, and a method of treating cancer by using the same. The concomitant use of the bispecific antibodies not only reduces the exhaustion of T cells, but also significantly facilitates tumor killing at a later stage. Moreover, the concomitant use of the bispecific antibodies, especially when targeting different epitopes of the same tumor-associated antigen or targeting different tumor-associated antigens, produces a strong synergistic killing effect. The use of the bispecific antibody combination and the pharmaceutical composition containing the same provides a potential clinical solution for effectively treating solid tumors.

EP 4 357 362 A1

FIG. 15

**Description**

The present application claims priority to Chinese Patent Application No. 202110678326X filed on June 18, 2021 and Chinese Patent Application No. 2022106340425 filed on June 6, 2022, the contents of which are incorporated herein by reference in their entireties.

## TECHNICAL FIELD

The present invention belongs to the field of biopharmaceuticals, and particularly relates to a bispecific antibody combination, a pharmaceutical composition containing the same and use thereof.

## BACKGROUND

Tumor cells can reduce the presentation of tumor antigens by down-regulating the major histocompatibility complex I (MHCI) on the cell surface, thereby evading the body's anti-tumor immune response. The CD3 bispecific antibody mediates the formation of immune synapses by simultaneously binding to CD3 molecules on T cells and to tumor-associated antigens on tumor cells, thereby killing the tumor cells. Theoretically, the CD3 bispecific antibody can directly activate CD3 molecules on T cells independent of the antigen presentation by the histocompatibility complex and activate polyclonal T cells independent of the activation of antigen-specific T cells, and thus has a strong effect of mediating T cells to kill tumor cells. In 2014, blinatumomab called BiTE by Amgen was marketed for treating recurrent/refractory B-cell acute lymphoma (B-ALL), and has achieved a good curative effect. Blinatumomab, as a bispecific antibody, binds to CD19 on tumor cells at one end, and binds to CD3 on T cells at the other end, and can mediate a strong tumor-killing effect by T cells; the initial response rate of patients exceeded 50% (Bejnjamin and Stein 2016, Ther Adv Hematol, 7(3):142-146).

CD3 bispecific antibodies account for nearly half of the bispecific antibodies in clinical trials underway, of which about 2/3 are directed against hematologic tumors and 1/3 are directed against solid tumors (Bispecific antibodies: a mechanistic review of the pipeline. Nature Review Drug Discovery, 2019 Aug; 18(8):585-608). Although the CD3 bispecific antibodies directed against solid tumors exhibit strong anti-tumor activity in experiments *in vitro* or in animal experiments, they have not yet achieved a satisfactory therapeutic effect in clinical studies/application. Solid tumors often contain a lot of stromal cells and extracellular matrix, the formed physical barrier blocks infiltration of lymphocytes, and there are only a small proportion of T cells in the solid tumors compared to the number of tumor cells; meanwhile, an internal immunosuppressive tumor microenvironment includes, for example, Tregs, MDSCs, TAMs, and immune checkpoints; and the CD3 bispecific antibody alone very likely causes T-cell apoptosis in the absence of the second signal in T cells. These factors may all contribute to poor clinical efficacy of CD3 bispecific antibodies in solid tumors. It has been reported in the literature that CD3 bispecific antibodies, in combination with a second signal such as 4-1BB, or with the integration of 4-1BB in the CAR intracellular segment of CAR-T cells, can significantly promote T-cell activation and reduce T-cell depletion, thereby improving the therapeutic efficacy (Transl. Med. 2019; 11, eaav5989).

B7-H4 (VTCN1, B7h.5, B7S1, B7x, or B7H4) is a transmembrane protein belonging to the B7/CD28 superfamily. B7-H4 protein is not expressed in most normal tissues or is expressed at a low level only in part of ductal epithelial cells of the body, such as breast ducts and lobules, oviduct epithelium, and endometrial glands. However, B7-H4 is overexpressed on the surfaces of tumor cells in breast cancer, ovarian cancer and endometrial cancer, particularly in triple negative breast cancer. Breast cancer is the second greatest malignancy worldwide with an increasing incidence. About one quarter of the female cancer patients are breast cancer patients. Ovarian cancer and endometrial cancer are common malignancies found in the female reproductive system. Ovarian cancer has the highest mortality rate and endometrial cancer has the third mortality rate among gynecological malignancies, and the development of a safer and more effective therapeutic approach is urgently needed. Given high expression of B7-H4 in this class of tumors and low expression thereof in normal tissues, the development of a bispecific antibody targeting B7-H4 is a promising therapeutic approach.

Therefore, it is of great importance to develop an antibody product that can provide a first signal and a second signal of T cells at the same time and can target tumors.

## SUMMARY

In order to address the lack of an effective bispecific antibody combination (namely a BsAb combination) in the prior art and to use it for the treatment of cancer, the present invention provides a BsAb combination, a pharmaceutical composition containing the same and use thereof.

In order to solve the above technical problems, a first technical solution of the present invention is as follows: provided is a BsAb combination, comprising a bispecific antibody I as a first therapeutic agent and a bispecific antibody II as a second therapeutic agent, wherein the bispecific antibody I comprises a CD3-targeting domain and a tumor-associated antigen (TAA)-targeting domain, and the bispecific antibody II comprises a 4-1BB-targeting domain and a TAA-targeting domain.

Preferably, in the bispecific antibody I, the TAA-targeting domain is a B7-H4-targeting domain; and/or, in the bispecific antibody II, the TAA-targeting domain is a B7-H4-targeting domain or a Her2-targeting domain.

More preferably, the B7-H4-targeting domains in the bispecific antibody I and the bispecific antibody II target different epitopes of B7-H4, respectively.

In some preferred embodiments, the bispecific antibody I is a Fab-Fc-scFv or Fab-Fc-(VH)$_n$ asymmetric structure, and the bispecific antibody II is an IgG-VH symmetric structure, wherein n is a natural number greater than or equal to 1.

Preferably, in the bispecific antibody I, the B7-H4-targeting domain is a scFv or VH_A-VH_B, wherein the VH_A and the VH_B are identical or different; the CD3-targeting domain is a Fab, wherein the Fab and the scFv are linked to an Fc via a hinge region or a linker peptide, or the Fab and the VH_A-VH_B are linked to an Fc via a hinge region or a linker peptide; the Fc is preferably an Fc of an IgG1, an amino acid sequence of the linker peptide is set forth in SEQ ID NOs: 93-96, 124, and more preferably, the Fc comprises an addition, deletion or mutation of 1-3 amino acids, such as mutations L234A and L235A, "knob" mutation T366W and "Hole" mutations T366S, L368A and Y407V, and/or, "knob" mutation S354C and "Hole" mutation Y349C; and/or,

in the bispecific antibody II, the B7-H4-targeting domain or the Her2-targeting domain is an IgG, and an Fc of the IgG preferably comprises an addition, deletion or mutation of 1-3 amino acids, such as mutations L234A and L235A; the 4-1BB-targeting domain is a VH or a scFv; the B7-H4 targeting domain or the Her2-targeting domain is linked to the 4-1BB-targeting domain via a linker peptide, and the amino acid sequence of the linker peptide is preferably set forth in SEQ ID NOs: 93-96.

In other preferred embodiments, in the bispecific antibody I, the B7-H4-targeting domain comprises a heavy chain variable region having amino acid sequences of HCDR1-3 set forth in SEQ ID NOs: 10, 21 and 33, respectively, and a light chain variable region having amino acid sequences of LCDR1-3 set forth in SEQ ID NOs: 43, 49 and 57, respectively; or the B7-H4-targeting domain comprises VH_A-VH_B, wherein the amino acid sequences of HCDR1-3 of the VH_A and the VH_B are both set forth in SEQ ID NOs: 100, 104 and 102, respectively, or, the amino acid sequences of HCDR1-3 of the VH_A are set forth in SEQ ID NOs: 100, 104 and 102, respectively, and amino acid sequences of HCDR1-3 of the VH_B are set forth in SEQ ID NOs: 97, 103 and 99, respectively; the CD3-targeting domain comprises a heavy chain variable region having amino acid sequences of HCDR1-3 set forth in SEQ ID NOs: 8, 19 and 31, respectively, and a light chain variable region having amino acid sequences of LCDR1-3 set forth in SEQ ID NOs: 41, 48 and 55, respectively; and/or,

in the bispecific antibody II, the B7-H4-targeting domain comprises a heavy chain variable region having amino acid sequences of HCDR1-3 set forth in SEQ ID NOs: 9, 20 and 32, respectively, and a light chain variable region having amino acid sequences of LCDR1-3 set forth in SEQ ID NOs: 42, 49 and 56, respectively; or, the heavy chain variable region has amino acid sequences of HCDR1-3 set forth in SEQ ID NOs: 10, 21 and 33, respectively, and the light chain variable region has amino acid sequences of LCDR1-3 set forth in SEQ ID NOs: 43, 49 and 57, respectively; the Her2-targeting domain comprises a heavy chain variable region having amino acid sequences of HCDR1-3 set forth in SEQ ID NOs: 6, 17 and 29, respectively, and a light chain variable region having amino acid sequences of LCDR1-3 set forth in SEQ ID NOs: 40, 47 and 54, respectively;

The 4-1BB-targeting domain is a VH having amino acid sequences of HCDR1-3 set forth in SEQ ID NOs: 7, 18 and 30 or SEQ ID NOs: 7, 22 and 30, respectively.

Preferably, the bispecific antibody II is selected from the group consisting of:

a) the bispecific antibody II comprises the B7-H4-targeting domain and the 4-1BB-targeting domain; wherein

in the B7-H4-targeting domain, the heavy chain variable region has amino acid sequences of HCDR1-3 set forth in SEQ ID NOs: 9, 20 and 32, respectively, and the light chain variable region has amino acid sequences of LCDR1-3 set forth in SEQ ID NOs: 42, 49 and 56, respectively; in the 4-1BB-targeting domain, the amino acid sequences of HCDR1-3 are set forth in SEQ ID NOs: 7, 22 and 30; or,
in the B7-H4-targeting domain, the heavy chain variable region has the amino acid sequences of HCDR1-3 set forth in SEQ ID NOs: 10, 21 and 33, respectively, and the light chain variable region has the amino acid sequences of LCDR1-3 set forth in SEQ ID NOs: 43, 49 and 57, respectively; in the 4-1BB-targeting domain, the amino acid sequences of HCDR1-3 are set forth in SEQ ID NOs: 7, 18 and 30; and

b) the bispecific antibody II comprises a Her2-targeting domain and a 4-1BB-targeting domain; wherein in the Her2-targeting domain, the heavy chain variable region has amino acid sequences of HCDR1-3 set forth in SEQ ID NOs: 6, 17 and 29, respectively, and the light chain variable region has amino acid sequences of LCDR1-3 set forth in SEQ ID NOs: 40, 47 and 54, respectively; in the 4-1BB-targeting domain, the amino acid sequences of HCDR1-3 are set forth in SEQ ID NOs: 7, 18 and 30, respectively.

In a specific embodiment, the BsAb combination is as follows: in the bispecific antibody I, the B7-H4-targeting domain comprises a heavy chain variable region having amino acid sequences of HCDR1-3 set forth in SEQ ID NOs: 10, 21 and 33, respectively, and a light chain variable region having amino acid sequences of LCDR1-3 set forth in SEQ ID NOs: 43, 49 and 57, respectively; the CD3-targeting domain comprises a heavy chain variable region having amino acid sequences of HCDR1-3 set forth in SEQ ID NOs: 8, 19 and 31, respectively, and a light chain variable region having amino acid sequences of LCDR1-3 set forth in SEQ ID NOs: 41, 48 and 55, respectively; in the bispecific antibody II, the B7-H4-targeting domain comprises a heavy chain variable region having amino acid sequences of HCDR1-3 set forth in SEQ ID NOs: 9, 20 and 32, respectively, and a light chain variable region having amino acid sequences of LCDR1-3 set forth in SEQ ID NOs: 42, 49 and 56, respectively; the 4-1BB-targeting domain has amino acid sequences of HCDR1-3 set forth in SEQ ID NOs: 7, 22 and 30, respectively. In another specific embodiment, the BsAb combination is as follows: in the bispecific antibody I, the B7-H4-targeting domain comprises a heavy chain variable region having amino acid sequences of HCDR1-3 set forth in SEQ ID NOs: 10, 21 and 33, respectively, and a light chain variable region having amino acid sequences of LCDR1-3 set forth in SEQ ID NOs: 43, 49 and 57, respectively; the CD3-targeting domain comprises a heavy chain variable region having amino acid sequences of HCDR1-3 set forth in SEQ ID NOs: 8, 19 and 31, respectively, and a light chain variable region having amino acid sequences of LCDR1-3 set forth in SEQ ID NOs: 41, 48 and 55, respectively; in the bispecific antibody II, the Her2-targeting domain comprises a heavy chain variable region having amino acid sequences of HCDR1-3 set forth in SEQ ID NOs: 6, 17 and 29, respectively, and a light chain variable region having amino acid sequences of LCDR1-3 set forth in SEQ ID NOs: 40, 47 and 54, respectively; the 4-1BB-targeting domain has amino acid sequences of HCDR1-3 set forth in SEQ ID NOs: 7, 18 and 30, respectively. In a specific embodiment, the BsAb combination is as follows: in the bispecific antibody I, the B7-H4-targeting domain comprises VH_A and VH_B having amino acid sequences of HCDR1-3 set forth in SEQ ID NOs: 100, 104 and 102, respectively, and the CD3-targeting domain comprises a heavy chain variable region having amino acid sequences of HCDR1-3 set forth in SEQ ID NOs: 8, 19 and 31, respectively, and a light chain variable region having amino acid sequences of LCDR1-3 set forth in SEQ ID NOs: 41, 48 and 55, respectively; in the bispecific antibody II, the B7-H4-targeting domain comprises a heavy chain variable region having amino acid sequences of HCDR1-3 set forth in SEQ ID NOs: 9, 20 and 32, respectively, and a light chain variable region having amino acid sequences of LCDR1-3 set forth in SEQ ID NOs: 42, 49 and 56, respectively; the 4-1BB-targeting domain has amino acid sequences of HCDR1-3 set forth in SEQ ID NOs: 7, 22 and 30, respectively.

In another specific embodiment, the BsAb combination is as follows: in the bispecific antibody I, the B7-H4-targeting domain comprises VH_A and VH_B having amino acid sequences of HCDR1-3 set forth in SEQ ID NOs: 100, 104 and 102, respectively, and the CD3-targeting domain comprises a heavy chain variable region having amino acid sequences of HCDR1-3 set forth in SEQ ID NOs: 8, 19 and 31, respectively, and a light chain variable region having amino acid sequences of LCDR1-3 set forth in SEQ ID NOs: 41, 48 and 55, respectively; in the bispecific antibody II, the Her2-targeting domain comprises a heavy chain variable region having amino acid sequences of HCDR1-3 set forth in SEQ ID NOs: 6, 17 and 29, respectively, and a light chain variable region having amino acid sequences of LCDR1-3 set forth in SEQ ID NOs: 40, 47 and 54, respectively; the 4-1BB-targeting domain has amino acid sequences of HCDR1-3 set forth in SEQ ID NOs: 7, 18 and 30, respectively.

In a specific embodiment, the BsAb combination is as follows: in the bispecific antibody I, the B7-H4-targeting domain comprises VH_A having amino acid sequences of HCDR1-3 set forth in SEQ ID NOs: 100, 104 and 102, respectively, and VH_B having amino acid sequences set forth in SEQ ID NOs: 97, 103 and 99, respectively, and the CD3-targeting domain comprises a heavy chain variable region having amino acid sequences of HCDR1-3 set forth in SEQ ID NOs: 8, 19 and 31, respectively, and a light chain variable region having amino acid sequences of LCDR1-3 set forth in SEQ ID NOs: 41, 48 and 55, respectively; in the bispecific antibody II, the B7-H4-targeting domain comprises a heavy chain variable region having amino acid sequences of HCDR1-3 set forth in SEQ ID NOs: 9, 20 and 32, respectively, and a light chain variable region having amino acid sequences of LCDR1-3 set forth in SEQ ID NOs: 42, 49 and 56, respectively; the 4-1BB-targeting domain has amino acid sequences of HCDR1-3 set forth in SEQ ID NOs: 7, 22 and 30, respectively.

In another specific embodiment, the BsAb combination is as follows: in the bispecific antibody I, the B7-H4-targeting domain comprises VH_A having amino acid sequences of HCDR1-3 set forth in SEQ ID NOs: 100, 104 and 102, respectively, and VH_B having amino acid sequences set forth in SEQ ID NOs: 97, 103 and 99, respectively, and the CD3-targeting domain comprises a heavy chain variable region having amino acid sequences of HCDR1-3 set forth in SEQ ID NOs: 8, 19 and 31, respectively, and a light chain variable region having amino acid sequences of LCDR1-3 set forth in SEQ ID NOs: 41, 48 and 55, respectively; in the bispecific antibody II, the Her2-targeting domain comprises a heavy chain variable region having amino acid sequences of HCDR1-3 set forth in SEQ ID NOs: 6, 17 and 29, respectively, and a light chain variable region having amino acid sequences of LCDR1-3 set forth in SEQ ID NOs: 40, 47 and 54, respectively; the 4-1BB-targeting domain has amino acid sequences of HCDR1-3 set forth in SEQ ID NOs:

7, 18 and 30, respectively.

In some preferred embodiments, in the bispecific antibody I, the B7-H4-targeting domain comprises a heavy chain variable region having an amino acid sequence set forth in SEQ ID NO: 66 and a light chain variable region having an amino acid sequence set forth in SEQ ID NO: 72; the CD3-targeting domain comprises a heavy chain variable region having an amino acid sequence set forth in SEQ ID NO: 67 and a light chain variable region having an amino acid sequence set forth in SEQ ID NO: 70; or, the B7-H4-targeting domain comprises a heavy chain variable region having an amino acid sequence set forth in SEQ ID NO: 66 and a light chain variable region having an amino acid sequence set forth in SEQ ID NO: 72; the CD3-targeting domain comprises a heavy chain variable region having an amino acid sequence set forth in SEQ ID NO: 64 and a light chain variable region having an amino acid sequence set forth in SEQ ID NO: 70; or, the B7-H4-targeting domain has an amino acid sequence set forth in SEQ ID NO: 122, and the CD3-targeting domain comprises a heavy chain variable region having an amino acid sequence set forth in SEQ ID NO: 67 and a light chain variable region having an amino acid sequence set forth in SEQ ID NO: 70; or, the B7-H4-targeting domain comprises an amino acid sequence set forth in SEQ ID NO: 123, and the CD3-targeting domain comprises a heavy chain variable region having an amino acid sequence set forth in SEQ ID NO: 64 and a light chain variable region having an amino acid sequence set forth in SEQ ID NO: 70;

and/or, the bispecific antibody II is selected from the group consisting of:

a) the bispecific antibody II comprises the B7-H4-targeting domain and the 4-1BB-targeting domain; wherein

in the B7-H4-targeting domain, the heavy chain variable region has an amino acid sequence set forth in SEQ ID NO: 65, and the light chain variable region has an amino acid sequence set forth in SEQ ID NO: 71; in the 4-1BB-targeting domain, the VH has an amino acid sequence set forth in SEQ ID NO: 68; or, in the B7-H4-targeting domain, the heavy chain variable region has an amino acid sequence set forth in SEQ ID NO: 66, and the light chain variable region has an amino acid sequence set forth in SEQ ID NO: 72; in the 4-1BB-targeting domain, the VH has an amino acid sequence set forth in SEQ ID NO: 63; and

b) the bispecific antibody II comprises a Her2-targeting domain and a 4-1BB-targeting domain; wherein in the Her2-targeting domain, the heavy chain variable region has an amino acid sequence set forth in SEQ ID NO: 62, and the light chain variable region has an amino acid sequence set forth in SEQ ID NO: 69; in the 4-1BB-targeting domain, the VH has an amino acid sequence set forth in SEQ ID NO: 63.

In a specific embodiment, the BsAb combination is as follows: in the bispecific antibody I, the B7-H4-targeting domain comprises a heavy chain variable region having an amino acid sequence set forth in SEQ ID NO: 66, and a light chain variable region having an amino acid sequence set forth in SEQ ID NO: 72; the CD3-targeting domain comprises a heavy chain variable region having an amino acid sequence set forth in SEQ ID NO: 67, and a light chain variable region having an amino acid sequence set forth in SEQ ID NO: 70; in the bispecific antibody II, the B7-H4-targeting domain comprises a heavy chain variable region having an amino acid sequence set forth in SEQ ID NO: 65, and a light chain variable region having an amino acid sequence set forth in SEQ ID NO: 71; the 4-1BB-targeting domain comprises a VH having an amino acid sequence set forth in SEQ ID NO: 68.

In another specific embodiment, the BsAb combination is as follows: in the bispecific antibody I, the B7-H4-targeting domain comprises a heavy chain variable region having an amino acid sequence set forth in SEQ ID NO: 66, and a light chain variable region having an amino acid sequence set forth in SEQ ID NO: 72; the CD3-targeting domain comprises a heavy chain variable region having an amino acid sequence set forth in SEQ ID NO: 67, and a light chain variable region having an amino acid sequence set forth in SEQ ID NO: 70; in the bispecific antibody II, the Her2-targeting domain comprises a heavy chain variable region having an amino acid sequence set forth in SEQ ID NO: 62, and a light chain variable region having an amino acid sequence set forth in SEQ ID NO: 69; the 4-1BB-targeting domain comprises a VH having an amino acid sequence set forth in SEQ ID NO: 63.

In a specific embodiment, the BsAb combination is as follows: in the bispecific antibody I, the B7-H4-targeting domain has an amino acid sequence set forth in SEQ ID NO: 122; the CD3-targeting domain comprises a heavy chain variable region having an amino acid sequence set forth in SEQ ID NO: 67, and a light chain variable region having an amino acid sequence set forth in SEQ ID NO: 70; in the bispecific antibody II, the B7-H4-targeting domain comprises a heavy chain variable region having an amino acid sequence set forth in SEQ ID NO: 65, and a light chain variable region having an amino acid sequence set forth in SEQ ID NO: 71; the 4-1BB-targeting domain comprises a VH having an amino acid sequence set forth in SEQ ID NO: 68.

In another specific embodiment, the BsAb combination is as follows: in the bispecific antibody I, the B7-H4-targeting domain has an amino acid sequence set forth in SEQ ID NO: 122; the CD3-targeting domain comprises a heavy chain variable region having an amino acid sequence set forth in SEQ ID NO: 67, and a light chain variable region having an amino acid sequence set forth in SEQ ID NO: 70; in the bispecific antibody II, the Her2-targeting domain comprises a heavy chain variable region having an amino acid sequence set forth in SEQ ID NO: 62, and a light chain variable region having an amino acid sequence set forth in SEQ ID NO: 69; the 4-1BB-targeting domain comprises a VH having an amino acid sequence set forth in SEQ ID NO: 63.

In a specific embodiment, the BsAb combination is as follows: in the bispecific antibody I, the B7-H4-targeting domain has an amino acid sequence set forth in SEQ ID NO: 123; the CD3-targeting domain comprises a heavy chain variable region having an amino acid sequence set forth in SEQ ID NO: 67, and a light chain variable region having an amino acid sequence set forth in SEQ ID NO: 70; in the bispecific antibody II, the B7-H4-targeting domain comprises a heavy chain variable region having an amino acid sequence set forth in SEQ ID NO: 65, and a light chain variable region having an amino acid sequence set forth in SEQ ID NO: 71; the 4-1BB-targeting domain comprises a VH having an amino acid sequence set forth in SEQ ID NO: 68.

In another specific embodiment, the BsAb combination is as follows: in the bispecific antibody I, the B7-H4-targeting domain has an amino acid sequence set forth in SEQ ID NO: 123; the CD3-targeting domain comprises a heavy chain variable region having an amino acid sequence set forth in SEQ ID NO: 67, and a light chain variable region having an amino acid sequence set forth in SEQ ID NO: 70; in the bispecific antibody II, the Her2-targeting domain comprises a heavy chain variable region having an amino acid sequence set forth in SEQ ID NO: 62, and a light chain variable region having an amino acid sequence set forth in SEQ ID NO: 69; the 4-1BB-targeting domain comprises a VH having an amino acid sequence set forth in SEQ ID NO: 63. In some preferred embodiments, in the bispecific antibody I, the B7-H4-targeting domain has amino acid sequences set forth in SEQ ID NOs: 92, 115 and 116, and the CD3-targeting domain comprises a heavy chain and a light chain having amino acid sequences set forth in SEQ ID NO: 78 and SEQ ID NO: 81, respectively; or, the B7-H4-targeting domain has amino acid sequences set forth in SEQ ID NOs: 92, 115 and 116, and the CD3-targeting domain comprises a heavy chain and a light chain having amino acid sequences set forth in SEQ ID NO: 75 and SEQ ID NO: 81, respectively; and/or,

in the bispecific antibody II, the B7-H4-targeting domain comprises a heavy chain and a light chain having amino acid sequences set forth in SEQ ID NO: 76 and SEQ ID NO: 82, or SEQ ID NO: 77 and SEQ ID NO: 83, respectively; the Her2-targeting domain comprises a heavy chain and a light chain having amino acid sequences set forth in SEQ ID NO: 73 and SEQ ID NO: 80, respectively; the 4-1BB-targeting domain comprises a heavy chain having an amino acid sequence set forth in SEQ ID NO: 74 or 79.

In a specific embodiment, the BsAb combination is as follows: in the bispecific antibody I, the B7-H4-targeting domain has the amino acid sequence set forth in SEQ ID NO: 92, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain has the amino acid sequence set forth in SEQ ID NO: 79; and the N-terminus of the 4-1BB-targeting domain is directly linked to the C-terminus of the heavy chain of the B7-H4-targeting domain.

In another specific embodiment, the BsAb combination is as follows: in the bispecific antibody I, the B7-H4-targeting domain has the amino acid sequence set forth in SEQ ID NO: 92, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain has the amino acid sequence set forth in SEQ ID NO: 79; and the N-terminus of the 4-1BB-targeting domain is linked to the C-terminus of the heavy chain of the B7-H4-targeting domain via the linker peptide as set forth in SEQ ID NO: 95.

In yet another specific embodiment, the BsAb combination is as follows: in the bispecific antibody I, the B7-H4-targeting domain has an amino acid sequence set forth in SEQ ID NO: 92, and the CD3-targeting domain comprises a heavy chain and a light chain having amino acid sequences set forth in SEQ ID NO: 78 and SEQ ID NO: 81, respectively; in the bispecific antibody II, the B7-H4-targeting domain comprises a heavy chain and a light chain having amino acid sequences set forth in SEQ ID NO: 73 and SEQ ID NO: 80, respectively, and the 4-1BB-targeting domain has an amino acid sequence set forth in SEQ ID NO: 74.

In a specific embodiment, the BsAb combination is as follows: in the bispecific antibody I, the B7-H4-targeting domain has the amino acid sequence set forth in SEQ ID NO: 115, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain has the amino acid sequence set forth in SEQ ID NO: 79; and the N-terminus of the 4-1BB-targeting domain is directly linked to the C-terminus of the heavy chain of the B7-H4-targeting domain.

In another specific embodiment, the BsAb combination is as follows: in the bispecific antibody I, the B7-H4-targeting domain has the amino acid sequence set forth in SEQ ID NO: 115, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain has the amino acid sequence set forth in SEQ ID NO: 79; and the N-terminus of the 4-1BB-targeting domain is linked to the C-terminus of the heavy chain of the B7-H4-targeting domain via the linker peptide as set forth in SEQ ID NO: 95.

In yet another specific embodiment, the BsAb combination is as follows: in the bispecific antibody I, the B7-H4-targeting domain has an amino acid sequence set forth in SEQ ID NO: 115, and the CD3-targeting domain comprises a heavy chain and a light chain having amino acid sequences set forth in SEQ ID NO: 78 and SEQ ID NO: 81, respectively; in the bispecific antibody II, the B7-H4-targeting domain comprises a heavy chain and a light chain having amino acid sequences set forth in SEQ ID NO: 73 and SEQ ID NO: 80, respectively, and the 4-1BB-targeting domain has an amino acid sequence set forth in SEQ ID NO: 74.

In a specific embodiment, the BsAb combination is as follows: in the bispecific antibody I, the B7-H4-targeting domain has the amino acid sequence set forth in SEQ ID NO: 116, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain has the amino acid sequence set forth in SEQ ID NO: 79; and the N-terminus of the 4-1BB-targeting domain is directly linked to the C-terminus of the heavy chain of the B7-H4-targeting domain.

In another specific embodiment, the BsAb combination is as follows: in the bispecific antibody I, the B7-H4-targeting domain comprises the amino acid sequence set forth in SEQ ID NO: 116, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain has the amino acid sequence set forth in SEQ ID NO: 79; and the N-terminus of the 4-1BB-targeting domain is linked to the C-terminus of the heavy chain of the B7-H4-targeting domain via the linker peptide as set forth in SEQ ID NO: 95.

In yet another specific embodiment, the BsAb combination is as follows: in the bispecific antibody I, the B7-H4-targeting domain has an amino acid sequence set forth in SEQ ID NO: 116, and the CD3-targeting domain comprises a heavy chain and a light chain having amino acid sequences set forth in SEQ ID NO: 78 and SEQ ID NO: 81, respectively; in the bispecific antibody II, the B7-H4-targeting domain comprises a heavy chain and a light chain having amino acid sequences set forth in SEQ ID NO: 73 and SEQ ID NO: 80, respectively, and the 4-1BB-targeting domain has an amino acid sequence set forth in SEQ ID NO: 74.

In order to solve the above technical problems, a second technical solution of the present invention is as follows: provided is a BsAb combination, comprising a bispecific antibody I and a bispecific antibody II, or a bispecific antibody I and urelumab, wherein the bispecific antibody I comprises 3 polypeptide chains: polypeptide chain-1, polypeptide chain-2, and polypeptide chain-3, the amino acid sequences of which are set forth in SEQ ID NO: 81, SEQ ID NO: 88, and SEQ ID NO: 87; or SEQ ID NO: 81, SEQ ID NO: 88, and SEQ ID NO: 87; or SEQ ID NO: 113, SEQ ID NO: 114, and SEQ ID NO: 115; or SEQ ID NO: 113, SEQ ID NO: 114, and SEQ ID NO: 116, respectively; and/or, the bispecific antibody II comprises 2 polypeptide chains: a short chain and a long chain, wherein amino acid sequences of the short chain and the long chain are set forth in SEQ ID NO: 82 and SEQ ID NO: 85; or SEQ ID NO: 82 and SEQ ID NO: 89; or SEQ ID NO: 82 and SEQ ID NO: 90; or SEQ ID NO: 83 and SEQ ID NO: 91; or SEQ ID NO: 80 and SEQ ID NO: 84, respectively.

In a specific embodiment, the BsAb combination is as follows: the bispecific antibody I comprises 3 polypeptide chains having the amino acid sequences set forth in SEQ ID NO: 81, SEQ ID NO: 88 and SEQ ID NO: 87, respectively; the bispecific antibody II comprises 2 polypeptide chains having the amino acid sequences set forth in SEQ ID NO: 82 and SEQ ID NO: 89, respectively.

In another specific embodiment, the BsAb combination is as follows: the bispecific antibody I comprises 3 polypeptide chains having the amino acid sequences set forth in SEQ ID NO: 81, SEQ ID NO: 88 and SEQ ID NO: 87, respectively; the bispecific antibody II comprises 2 polypeptide chains having the amino acid sequences set forth in SEQ ID NO: 82 and SEQ ID NO: 85, respectively.

In yet another specific embodiment, the BsAb combination is as follows: the bispecific antibody I comprises 3 polypeptide chains having the amino acid sequences set forth in SEQ ID NO: 81, SEQ ID NO: 88 and SEQ ID NO: 87, respectively; the bispecific antibody II comprises 2 polypeptide chains having the amino acid sequences set forth in SEQ ID NO: 80 and SEQ ID NO: 84, respectively.

In a specific embodiment, the BsAb combination is as follows: the bispecific antibody I comprises 3 polypeptide chains having the amino acid sequences set forth in SEQ ID NO: 113, SEQ ID NO: 114 and SEQ ID NO: 115, respectively; the bispecific antibody II comprises 2 polypeptide chains having the amino acid sequences set forth in SEQ ID NO: 82 and SEQ ID NO: 89, respectively.

In another specific embodiment, the BsAb combination is as follows: the bispecific antibody I comprises 3 polypeptide chains having the amino acid sequences set forth in SEQ ID NO: 113, SEQ ID NO: 114 and SEQ ID NO: 115, respectively; the bispecific antibody II comprises 2 polypeptide chains having the amino acid sequences set forth in SEQ ID NO: 82 and SEQ ID NO: 85, respectively.

In yet another specific embodiment, the BsAb combination is as follows: the bispecific antibody I comprises 3 polypeptide chains having the amino acid sequences set forth in SEQ ID NO: 113, SEQ ID NO: 114 and SEQ ID NO: 115, respectively; the bispecific antibody II comprises 2 polypeptide chains having the amino acid sequences set forth in SEQ ID NO: 80 and SEQ ID NO: 84, respectively.

In a specific embodiment, the BsAb combination is as follows: the bispecific antibody I comprises 3 polypeptide chains having the amino acid sequences set forth in SEQ ID NO: 113, SEQ ID NO: 114 and SEQ ID NO: 116, respectively; the bispecific antibody II comprises 2 polypeptide chains having the amino acid sequences set forth in SEQ ID NO: 82 and SEQ ID NO: 89, respectively.

In another specific embodiment, the BsAb combination is as follows: the bispecific antibody I comprises 3 polypeptide chains having the amino acid sequences set forth in SEQ ID NO: 113, SEQ ID NO: 114 and SEQ ID NO: 116, respectively; the bispecific antibody II comprises 2 polypeptide chains having the amino acid sequences set forth in SEQ ID NO: 82 and SEQ ID NO: 85, respectively.

In yet another specific embodiment, the BsAb combination is as follows: the bispecific antibody I comprises 3 polypeptide chains having the amino acid sequences set forth in SEQ ID NO: 113, SEQ ID NO: 114 and SEQ ID NO: 116, respectively; the bispecific antibody II comprises 2 polypeptide chains having the amino acid sequences set forth in SEQ ID NO: 80 and SEQ ID NO: 84, respectively.

In order to solve the above technical problems, a third technical solution of the present invention is as follows: provided is an isolated nucleic acid, wherein the isolated nucleic acid encodes the bispecific antibody I and the bispecific antibody II of the BsAb combination according to the first or second technical solution, respectively.

In order to solve the above technical problems, a fourth technical solution of the present invention is as follows: provided is a recombinant expression vector, comprising the isolated nucleic acid according to the third technical solution.

In order to solve the above technical problems, a fifth technical solution of the present invention is as follows: provided is a transformant, comprising the isolated nucleic acid according to the third technical solution or the recombinant expression vector according to the fourth technical solution; preferably, the host of the transformant is a prokaryotic cell, preferably an escherichia coli cell, or a eukaryotic cell, preferably a yeast cell or a mammalian cell.

In order to solve the above technical problems, a sixth technical solution of the present invention is as follows: provided is a pharmaceutical composition, comprising the BsAb combination according to the first or second technical solution.

Preferably, the pharmaceutical composition further comprises a third therapeutic agent, such as an immune checkpoint antibody and/or a chemotherapeutic agent; and optionally a pharmaceutically acceptable carrier.

The pharmaceutically acceptable carrier may be a conventional carrier in the art, and the carrier may be any suitable physiologically or pharmaceutically acceptable auxiliary material. The pharmaceutically acceptable auxiliary material is one conventional in the art, and preferably comprises a pharmaceutically acceptable excipient, a filler, a diluent, or the like. More preferably, the pharmaceutical composition comprises 0.01%-99.99% of the above bispecific antibody combination, and 0.01%-99.99% of a pharmaceutically acceptable carrier, the percentage being the mass percentage of the pharmaceutical composition.

The route of administration for the pharmaceutical composition described herein is preferably parenteral administration, injection administration or oral administration. The injection administration preferably includes intravenous injection, intramuscular injection, intraperitoneal injection, intradermal injection, subcutaneous injection or the like. The pharmaceutical composition is in any conventional dosage form in the art, preferably in the form of a solid, semisolid or liquid, i.e., it may be an aqueous solution, a non-aqueous solution or a suspension, more preferably a tablet, capsule, granule, injection, infusion, or the like. More preferably, it is administered intravascularly, subcutaneously, intraperitoneally or intramuscularly. Preferably, the pharmaceutical composition may also be administered as an aerosol or a coarse spray, i.e., administered nasally; or administered intrathecally, intramedullarily or intraventricularly. More preferably, the pharmaceutical composition may also be administered transdermally, percutaneously, topically, enterally, intravaginally, sublingually or rectally. The pharmaceutical composition of the present invention may be formulated into various dosage forms as required, and can be administered by a physician in the light of the patient's type, age, weight, and general disease state, route of administration, etc. The administration may be performed, for example, by injection or other therapeutic modalities.

The dose level at which the pharmaceutical composition of the present invention is administered can be adjusted depending on the amount of the composition to achieve the desired diagnostic or therapeutic outcome. The administration regimen may also be a single injection or multiple injections, or an adjusted one. The selected dose level and regimen is appropriately adjusted depending on a variety of factors including the activity and stability (i.e., half-life) of the pharmaceutical composition, the formulation, the route of administration, combination with other drugs or treatments, the disease or disorder to be detected and/or treated, and the health condition and previous medical history of the subject to be treated.

A therapeutically effective dose for the pharmaceutical composition of the present invention may be estimated initially in cell culture experiments or animal models such as rodents, rabbits, dogs, pigs and/or primates. Animal models can also be used to determine the appropriate concentration range and route of administration, and subsequently an effective dose and a route of administration in humans. In general, the determination of and adjustment to the effective amount or dose to be administered and the assessment of when and how to make such adjustments are known to those skilled in the art.

For combination therapy, the above antibody, the bispecific antibody, and/or additional therapeutic or diagnostic agents may each be used as a single agent for use within any time frame suitable for performing the intended treatment or diagnosis. Thus, these single agents may be administered substantially simultaneously (i.e., as a single formulation or within minutes or hours) or sequentially.

For additional guidance regarding formulations, doses, dosage regimens, and measurable therapeutic outcomes, see Berkow et al. (2000) The Merck Manual of Medical Information and Merck & Co. Inc., Whitehouse Station, New Jersey; Ebadi (1998) CRC Desk Reference of Clinical Pharmacology, etc.

In order to solve the above technical problems, a seventh technical solution of the present invention is as follows: provided is a kit, comprising the BsAb combination according to the first or second technical solution, the isolated nucleic acid according to the third technical solution, the recombinant expression vector according to the fourth technical solution, the transformant according to the fifth technical solution, or the pharmaceutical composition according to the sixth technical solution, and optionally instructions.

In order to solve the above technical problems, an eighth technical solution of the present invention is as follows: provided is use of the BsAb combination according to the first or second technical solution, the isolated nucleic acid according to the third technical solution, the recombinant expression vector according to the fourth technical solution, the transformant according to the fifth technical solution, or the pharmaceutical composition according to the sixth technical solution in the preparation of a medicament for treating cancer.

Preferably, the cancer is a hematologic tumor or a solid tumor, and the solid tumor is preferably breast cancer, ovarian cancer, or endometrial cancer.

In order to solve the above technical problems, a ninth technical solution of the present invention is as follows: provided is a kit of parts, comprising a kit I and a kit II,

wherein the kit I comprises the bispecific antibody I of the BsAb combination according to the first or second technical solution, and the kit II comprises the bispecific antibody II of the BsAb combination according to the first or second technical solution; or

the kit I comprises the bispecific antibody I and the bispecific antibody II of the BsAb combination according to the first or second technical solution; the kit II comprises a third therapeutic agent such as an immune checkpoint antibody and/or a chemotherapeutic agent.

In order to solve the above technical problems, a tenth technical solution of the present invention is as follows: provided is an administration device, comprising: (1) an infusion module for administering to a subject in need thereof the pharmaceutical composition according to the sixth technical solution, and (2) optionally a pharmacodynamic monitoring module.

The infusion module comprises an infusion device and/or an injection device, such as an infusion pump or a syringe, for use in continuous, intermittent or timed delivery of a liquid drug, such as an antibody or an antibody combination, such as a BsAb combination, or a pharmaceutical composition, or a chemotherapeutic agent, to a subject in need thereof. For example, the infusion pump is capable of accurately controlling the number of infusion drops or the flow rate of the infusion, ensuring that the antibody combination or the pharmaceutical composition is able to safely enter the body of a subject in need thereof at a uniform rate and with an accurate dose. The pharmacodynamic monitoring module comprises a data processing unit, an infusion monitoring unit, and an infusion control unit. The data processing unit receives and evaluates common information associated with the treatment, such as pharmacodynamics, and pharmacokinetics, for evaluating drug efficacy, safety, and/or compliance; in certain embodiments, pharmacodynamics is represented by a curve or function describing the percentage (or amount) of drug "activity" in the blood within a certain time period after delivery of a large dose of the drug. The infusion monitoring unit and the infusion control unit are connected with the infusion module and are respectively used for acquiring and controlling the infusion amount of the infusion module.

The pharmacodynamic monitoring module optionally further comprises one or more of a nursing record reading unit, a liquid output monitoring unit, a water supply monitoring unit, a warning device and a display device which are connected to the data processing unit. The nursing record reading unit is connected with the nursing system and is used for acquiring the amount of a liquid drug dosed to a patient via opisthenar injection; the water supply monitoring unit is connected with the water supply device and is used for acquiring the water supply for a subject in need; the liquid output monitoring unit is connected with the discharge receiving device and is used for acquiring the liquid output for a subject in need; the warning device is used for generating warnings according to the processing result of the data processing unit; the display device is used for displaying infusion information, liquid drug amount, micturition volume, water supply and/or perspiration amount of a subject in need and the processing result of the data processing unit according to the infusion information, liquid medicine amount, micturition volume, water supply and perspiration amount.

In order to solve the above technical problems, an eleventh technical solution of the present invention is as follows: provided is a method for treating cancer, comprising administering to a subject in need thereof the BsAb combination according to the first or second technical solution, the pharmaceutical composition according to the sixth technical solution, the kit of parts according to the ninth technical solution, or the administration device according to the tenth technical solution.

Preferably, the cancer is a hematologic tumor or a solid tumor, and the solid tumor is preferably breast cancer, ovarian cancer, or endometrial cancer.

Preferably, the bispecific antibody I and the bispecific antibody II of the BsAb combination are administered simultaneously or sequentially.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

The three-letter codes and single-letter codes for amino acids used in the present invention are known to those skilled in the art, or are described in J. Biol. Chem, 243, p3558 (1968).

As used herein, the term "include/includes/including" or "comprise/comprises/comprising" is intended to mean that a composition and a method include the elements described but does not exclude other elements; but the case of "consist/consists/consisting of" is also included as the context dictates.

The term "antibody" used in the present invention includes an immunoglobulin (Ig), which is a tetrapeptide chain structure formed by linking two identical heavy chains to two identical light chains via interchain disulfide bonds. Immunoglobulins differ in amino acid composition and arrangement of their heavy chain constant regions and therefore in their antigenicity. Accordingly, immunoglobulins can be classified into five classes, or isotypes of immunoglobulins, namely IgM, IgD, IgG, IgA and IgE, with their corresponding heavy chains being the $\mu$, $\delta$, $\gamma$, $\alpha$ and $\epsilon$ chains, respectively. The Ig of the same class can be divided into different subclasses according to the differences in amino acid composition of the hinge regions and the number and location of disulfide bonds in the heavy chains; for example, IgG can be divided into IgG1, IgG2, IgG3, and IgG4. Light chains are classified into $\kappa$ or $\lambda$ chains by the difference in the constant regions. Each of the five classes of Ig may have a $\kappa$ chain or a $\lambda$ chain. In the present invention, the light chain variable region of the antibody of the present invention may further comprise a light chain constant region comprising a human $\kappa$ or $\lambda$ chain or a variant thereof. In the present invention, the heavy chain variable region of the antibody of the present invention may further comprise a heavy chain constant region comprising human IgG1, IgG2, IgG3, IgG4 or a variant thereof.

The sequences of about 110 amino acids of the heavy and light chains of the antibody near the N-terminus vary considerably and thus are referred to as variable regions (V regions); the remaining amino acid sequences near the C-terminus are relatively stable and thus are referred to as constant regions (C regions). Each of the light chain variable regions (VLs) and the heavy chain variable region (VHs) consists of 3 complementary determining regions (CDRs) and 4 framework regions (FWRs) arranged from the amino-terminus to the carboxyl-terminus in the following order: FWR1, CDR1, FWR2, CDR2, FWR3, CDR3, and FWR4. The 3 CDRs of the light chain refer to LCDR1, LCDR2, and LCDR3; the 3 CDRs of the heavy chain refer to HCDR1, HCDR2 and HCDR3.

It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. In the present invention, the amino acid sequences of the listed CDRs are shown according to the Chothia scheme. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-948, 1997). Although the scope claimed in the present invention is the sequences shown based on the Chothia scheme, the amino acid sequences corresponding to the other schemes for numbering CDRs shall also fall within the scope of the present invention.

The term "mutation" includes substitutions, additions and/or deletions of amino acids or nucleotides. The "amino acid substitution" is replacement of an amino acid residue by another amino acid residue and replacement by an amino acid residue with similar side chains. In the present invention, the mutation may include mutations currently known to those skilled in the art, for example, mutations that may be made to an antibody during the production or application of the antibody, for example, mutations made to sites that may be present, especially those subjected to potential post-transcriptional modifications (PTMs) in CDRs, including aggregation of antibodies, asparagine deamidation (NG, NS, NH, etc.) sensitive sites, aspartate isomerization (DG, DP) sensitive sites, N-glycosylation (N-{P}S/T) sensitive sites, oxidation sensitive sites, and the like. In the present invention, an antibody with an amino acid mutation is referred to as a variant.

The term "vector" or "expression vector" used herein is a composition that comprises an isolated nucleic acid and is useful for delivering the isolated nucleic acid to the interior of a cell. Many vectors are known in the art, including but not limited to, linear polynucleotides, polynucleotides associated with ionic or amphiphilic compounds, plasmids, and viruses. Thus, the term "vector" includes autonomously replicating plasmids or viruses. The term should also be construed as including non-plasmid and non-viral compounds that facilitate the transfer of nucleic acids into cells, such as polylysine compounds and liposomes. Examples of viral vectors include, but are not limited to, adenoviral vectors, adeno-associated viral vectors, retroviral vectors, etc.

The term "transfection" refers to the introduction of an exogenous nucleic acid into a eukaryotic cell. Transfection may be accomplished by a variety of means known in the art, including calcium phosphate-DNA co-precipitation, DEAE-dextran mediated transfection, polybrene-mediated transfection, electroporation, microinjection, liposome fusion, lipofection, protoplast fusion, retroviral infection, and biolistics.

As used herein, the term "$EC_{50}$" refers to the concentration for 50% of maximal effect, i.e., the concentration that can cause 50% of the maximal effect.

As used herein, the terms "cancer" and "tumors" are intended to include all types of cancerous growths or oncogenic processes, metastatic tissues or malignantly transformed cells, tissues or organs, regardless of their histopathological types or stages of invasiveness. Examples include, but are not limited to, solid tumors, hematologic cancer, soft tissue tumors and metastatic lesions.

On the basis of the general knowledge in the art, the above preferred conditions can be combined arbitrarily to obtain preferred embodiments of the present invention.

The reagents and starting materials used in the present invention are commercially available. The beneficial effects of the present invention are as follows:

1) The BsAb combination of the present invention is a combination of TAA×CD3 bispecific antibody I and TAA×4-1BB bispecific antibody II, preferably a combination of B7-H4×CD3 bispecific antibody, and TAA×4-1BB bispecific antibody such as B7-H4 (or Her2)×4-1BB bispecific antibody. The combination use of B7-H4×CD3 bispecific antibody and B7-H4×4-1BB bispecific antibody, such as the combination use of bispecific antibodies PR003899 and PR004281, not only reduces T-cell depletion, but also can significantly promote tumor killing in the later stage; the bispecific antibody combination use of PR007168 and PR004282 can further enhance the effect of killing tumor cells.

2) In the present invention, the bispecific antibody TAA×CD3 (preferably B7-H4×CD3) such as PR003899 alone does not have any killing effect at a low ratio of effector cells to target cells, while the combination use of TAA×CD3 and TAA×4-1BB (preferably B7-H4×4-1BB) shows a strong killing effect. Especially, the combination use of TAA×CD3 and PR003338 of different epitopes of the same TAA or PR002828 of different TAAs exhibits a strong synergistic effect; the bispecific antibody combination of PR007078 and PR004282 significantly promotes division and proliferation of T cells and reduces apoptosis of T cells.

Thus, the use of the BsAb combination of the present invention provides a potential and clinically effective solution for treating solid tumors.

## BRIEF DESCRIPTION OF THE DRAWINGS

FIG. 1 shows the structure and design of B7-H4×CD3 (Fab-Fc-ScFv), B7-H4×4-1BB (IgG-HC-VH) and Her2×4-1BB (IgG-HC-VH) bispecific antibodies;

FIG. 2 shows an assay for the binding of B7-H4×CD3 bispecific antibodies PR003733 and PR003899 to human, monkey and murine B7-H4 using flow cytometry;

FIG. 3 shows an assay for the binding of B7-H4×CD3 bispecific antibodies PR003733 and PR003899 to B7-H4 on tumor cells and to T cells using flow cytometry;

FIG. 4 shows the use of B7-H4×CD3 bispecific antibodies PR003733 and PR003899 in mediating T cells for killing MDA-MB-468 tumor cells;

FIG. 5 shows the use of B7-H4×CD3 bispecific antibodies PR003733 and PR003899 in mediating T cells for killing HCC-1954 tumor cells;

FIG. 6 shows the use of B7-H4×CD3 bispecific antibodies PR003733 and PR003899 in mediating T cells for killing BT-474 tumor cells;

FIG. 7 shows the binding of B7-H4×4-1BB bispecific antibodies PR004281 and PR004282 to human and monkey 4-1BB;

FIG. 8 shows the binding of B7-H4×4-1BB bispecific antibodies PR004281 and PR004282 to human, monkey and murine B7-H4 and to B7-H4 on tumor cells;

FIG. 9 shows an assay for *in-vitro* activation of T cells by B7-H4×4-1BB bispecific antibodies PR004281 and PR 004282;

FIG. 10 shows IHC staining of a human breast cancer tissue section to detect the ratio of T cells to tumor cells;

FIG. 11 shows an *in-vitro* killing assay for B7-H4×CD3 bispecific antibody molecules PR003733 and PR003899 at a low ratio of T cells to tumor cells;

FIG. 12 shows the combination use of B7-H4×CD3 (PR003899) and B7-H4×4-1BB (PR004281) in reducing T-cell depletion;

FIG. 13 shows the combination use of B7-H4×CD3 (PR003899) and B7-H4×4-1BB (PR004281) in promoting tumor killing;

FIG. 14 shows the combination use of B7-H4×CD3 (PR003899) and B7-H4×4-1BB (PR004359, PR003338, and PR002828) in promoting tumor killing;

FIG. 15 shows an *in-vitro* killing assay for the combination of different BsAb combinations (PR003899 and PR004359, PR003899 and PR003338, PR003899 and PR002828);

FIG. 16 shows a schematic diagram of the asymmetric molecular structure of B7-H4×CD3 (Fab-Fc-VH-VH);

FIG. 17 shows an assay for the binding of B7-H4×CD3 bispecific antibodies PR007078 and PR007168 to human, monkey and murine B7-H4 using flow cytometry;

FIG. 18 shows an assay for the binding of B7-H4×CD3 bispecific antibodies PR007078 and PR007168 to B7-H4 on tumor cells and to T cells using flow cytometry;

FIG. 19 shows the use of B7-H4×CD3 bispecific antibodies PR007078 and PR007168 in mediating T cells for killing MDA-MB-468 tumor cells;

FIG. 20 shows the use of B7-H4×CD3 bispecific antibodies PR007078 and PR007168 in mediating T cells for killing HCC-1954 tumor cells;

FIG. 21 shows the combination use of B7-H4×CD3 (PR007078 and PR007168) and B7-H4×4-1BB (PR004282) in mediating T cells for killing SK-BR-3 tumor cells;

FIG. 22 shows the combination use of B7-H4×CD3 (PR007078 and PR007168) and B7-H4×4-1BB (PR004282) in increasing T-cell proliferation;

FIG. 23 shows the combination use of B7-H4×CD3 (PR007078 and PR007168) and B7-H4×4-1BB (PR004282) in increasing T-cell division;

FIG. 24 shows the combination use of B7-H4×CD3 (PR007078 and PR007168) and B7-H4×4-1BB (PR004282) in reducing T-cell apoptosis.

## DETAILED DESCRIPTION

The present invention is further illustrated by the following examples, which are not intended to limit the present invention. Experimental procedures without specified conditions in the following examples are performed in accordance with conventional procedures and conditions, or in accordance with instructions.

Example 1: Structure and Design of Bispecific Antibodies

1.1. Construction of bispecific antibody I with Fab-Fc-scFv asymmetric structure (Structure No. A)

The bispecific antibody I (B7-H4×CD3 bispecific antibody molecule) comprises two domains, one of which can recognize B7-H4 specifically expressed on the surface of tumor cells, and the other of which can bind to CD3 molecules on T cells. After binding to the surface of tumor cells, the B7-H4×CD3 bispecific antibody molecules can recruit and activate T cells in the vicinity of tumor cells, thereby killing the tumor cells.

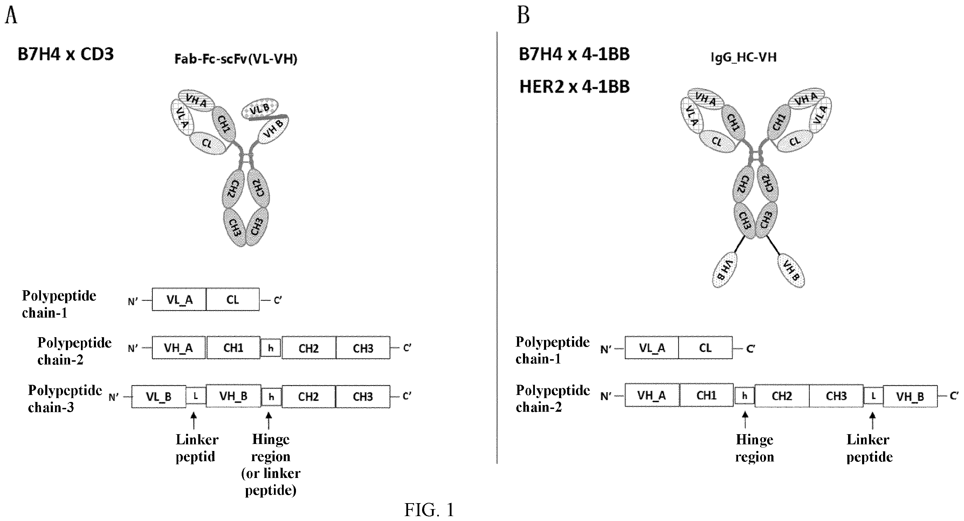

A of FIG. 1 is a schematic diagram of the asymmetric molecular structure of B7-H4×CD3 (Fab-Fc-scFv), wherein the B7-H4-targeting domain is a scFv structure, the CD3-targeting domain is a Fab structure, and the bispecific antibody molecule comprises three polypeptide chains: polypeptide chain-1, polypeptide chain-2, and polypeptide chain-3. From the N-terminus to the C-terminus, the polypeptide chain-1 has a structure of VLA-CL, the polypeptide chain-2 has a structure of VH_A-CH1-h-CH2-CH3, and the polypeptide chain-3 has a structure of VL_B - L-VH_B- h-CH2-CH3, wherein A and B represent targeting different targets, L is a linker peptide, and h is a hinge region.

To minimize the formation of byproducts with mismatched heavy chains (e.g., mismatching of two heavy chains of the anti-CD3 antibody), a mutant heterodimeric Fc region carrying a "knob-hole" mutation and a modified disulfide bond was used, as described in WO2009080251 and WO2009080252. The B7-H4×CD3 bispecific antibody has an Fc of IgG1 and carries mutations L234A and L235A (numbered according to the EU index) on CH2 of the Fc. Bispecific antibodies were generated by co-transfecting simultaneously three different mammalian expression vectors encoding: 1) the ScFv heavy chain of the corresponding antiB7-H4 antibody, which carries a "Hole" mutation in the Fc region so as to produce a heterodimeric antibody, CH2 of the Fc carrying mutations L234A and L235A; 2) the heavy chain of the corresponding anti-CD3 antibody, which carries a "knob" mutation in the Fc region so as to produce a heterodimeric antibody, CH2 of the Fc carrying mutations L234A and L235A; and 3) the light chain of the corresponding anti-CD3 antibody. The "knob" mutation in the Fc region of human IgG1 consists of: T366W, and the "Hole" mutation consists of: T366S, L368A, and Y407V In addition, S354C in the "knob" Fc region and "Hole" Y349C may be included; they form a pair of disulfide bonds to increase the stability and the yield of the heterodimeric antibody.

1.2. Construction of bispecific antibody I with Fab-Fc-VH-VH asymmetric structure (Structure No. C)

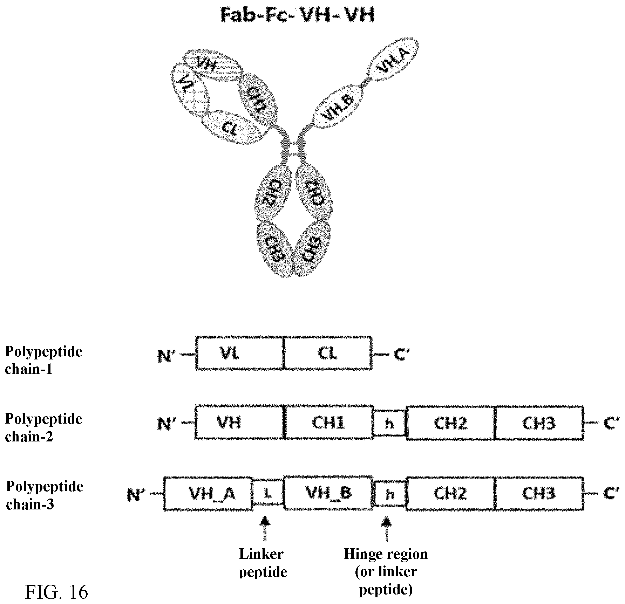

FIG. 16 is a schematic diagram of the asymmetric molecular structure of B7-H4×CD3, wherein the bispecific antibody molecule comprises three polypeptide chains: polypeptide chain-1, polypeptide chain-2, and polypeptide chain-3. The polypeptide chain-1 and the polypeptide chain-2 constitute the Fab portion targeting CD3, and the polypeptide chain-3 comprises two VH portions targeting B7-H4 (VH_A-VH_B). The sequences of VH_A and VH_B can be identical or different, and VH_A and VH_B are linked via a linker peptide GS _15.

To prevent crosslinking and reduced effector functions caused by Fcγ receptor binding, "AA" double mutations (L234A and L235A) or "AAA" triple mutants (L234A, L235A and G237A) were introduced into the heavy chain constant regions of the polypeptide chain-2 and the polypeptide chain-3. Furthermore, to reduce the production of heavy chain homodimers, different amino acid mutations were introduced into the constant regions of the two heavy chains, respectively, such that they could carry a "knob-hole" mutation and a modified disulfide bond. Mutations T366W and S354C were introduced into the polypeptide chain-2 targeting CD3; meanwhile, mutations T366S, L368A, Y407V and Y349C were introduced into the polypeptide chain-3 targeting B7-H4. Furthermore, to improve the thermostability of the antibody molecule, B7-H4 mutated two amino acids at positions 49 and 69 (according to the Kabat numbering system) in the VH domain of anti-B7-H4 antibody into Cys, making them form a disulfide bond; namely, mutations S49C and I70C were introduced, respectively.

1.3. Construction of bispecific antibody II with IgG-VH tetravalent symmetric structure (Structure No. B)

The bispecific antibody II is a TAA×4-1BB bispecific antibody, and the TAA is only exemplified herein by B7-H4 and Her2, and is intended to promote 4-1BB aggregation by bridging T cells with TAA positive tumor cells, so as to provide effective co-stimulatory signals for tumor antigen-specific T cells, thereby enhancing T cell receptor (TCR)-mediated activity and causing tumor lysis. Therefore, TAA×4-1BB-mediated activation of 4-1BB is orientated toward co-localization of T cells and tumor cells *in vivo*, reducing toxic response brought by peripheral activation.

B of FIG. 1 is a schematic diagram of the symmetric molecular structure of TAA×4-1BB (IgG-VH), wherein the domain of IgG-VH tetravalent symmetric structure comprises two polypeptide chains: polypeptide chain 1, also known as a short chain, comprising, from the amino-terminus to the carboxyl-terminus, VL_A-CL; and polypeptide chain 2, also known as a long chain, comprising, from the amino-terminus to the carboxyl-terminus, VH_A-CH1-h-CH2-CH3-L-VH_B; wherein A and B represent targeting different targets, L is a linker peptide, and h is a hinge region. The TAA×4-1BB bispecific antibody molecule with the symmetric structure can specifically recognize tumor cells through an IgG at the N-terminus, and recognize and activate T cells through a VH at the C-terminus. Moreover, the functions of the Fc are removed by introducing mutations L234A and L235A, reducing non-specific activation of 4-1BB on the periphery, and realizing specific killing of TAA×4-1BB bispecific antibody on tumor cells. Examples of TAA×4-1BB include PR004281, PR004282, PR004359, PR003338, and PR002828.

The structural information of the B7-H4×CD3 bispecific antibody I of the present invention is shown in Table 3 below.

The structural information of the TAA×4-1BB (B7-H4×4-1BB, HER2×4-1BB) bispecific antibody II of the present invention is shown in Table 4 below.

1.4. Antibody information

The information of the control molecules used herein and the parent monoclonal antibodies (mAb) used in the construction of the bispecific antibody molecules is shown in Table 1 below. The sequence numbers of the control molecules and the parent monoclonal antibodies in the sequence list are shown in Table 2 below.

Table 1. Information of control molecules or parent monoclonal antibodies

| Antibody No. | Antibody |
|---|---|
| PR002408 | Anti-B7-H4 1025_80C8-2E9 (H: G55A; L: N92Q), hIgG1 |
| PR002410 | Anti-B7-H4 1025_B-1H11 (L: C87Y), hIgG1 |
| PR003366 | Variant molecule of scFv-Fc form of PR002410 |
| PR001838 | Anti-4-1BB HCAb mAb 1016P0030B2, hIgG1 |
| PR004469 | Variant molecule of PR001838, carrying mutation G53A |
| PR000210 | Anti-HER2 trastuzumab analog, hIgG1 |
| PR001848 | Anti-CD3 humanized antibody, from WO2021063330 |
| PR003886 | Anti-CD3 humanized antibody, from WO2021063330 |
| PR006004 | Anti-B7-H4 humanized antibody |
| PR006008 | Anti-B7-H4 humanized antibody |
| PR007440 | PTM variant (G55S) of PR006004, IgG1 |
| PR007441 | PTM variant (G55S) of PR006008, IgG1 |

Table 2. Sequence numbers (SEQ ID NOs) of control molecules or parent monoclonal antibodies

| Antibody No. | Light chain | Heavy chain | VL | VH | LCDR1 | LCDR2 | LCDR3 | HCDR1 | HCDR2 | HCDR3 |
|---|---|---|---|---|---|---|---|---|---|---|
| PR002408 | 82 | 76 | 71 | 65 | 42 | 49 | 56 | 9 | 20 | 32 |
| PR002410 | 83 | 77 | 72 | 66 | 43 | 49 | 57 | 10 | 21 | 33 |
| PR003366 | | 92 | 72 | 66 | 43 | 49 | 57 | 10 | 21 | 33 |
| PR000210 | 80 | 73 | 69 | 62 | 40 | 47 | 54 | 6 | 17 | 29 |
| PR001848 | 81 | 75 | 70 | 64 | 41 | 48 | 55 | 8 | 19 | 31 |
| PR003886 | 81 | 78 | 70 | 67 | 41 | 48 | 55 | 8 | 19 | 31 |
| PR001838 | | 74 | | 63 | | | | 7 | 18 | 30 |
| PR004469 | | 79 | | 68 | | | | 7 | 22 | 30 |
| PR006004 | | 105 | | 106 | | | | 97 | 98 | 99 |
| PR006008 | | 107 | | 108 | | | | 100 | 101 | 102 |
| PR007440 | | 109 | | 110 | | | | 97 | 103 | 99 |
| PR007441 | | 111 | | 112 | | | | 100 | 104 | 102 |

Table 3. Structure of B7-H4×CD3 bispecific antibody I

| Structure No. | Bispecific antibody molecules | CD3 antibody | B7-H4 antibody | Structure of CD3 end | Structure of B7-H4 end | Linker peptide | Fc type of CD3 end (mutation) | Fc type of B7-H4 end (mutation) |
|---|---|---|---|---|---|---|---|---|
| A | PR003899 | PR003886 | PR003366 | Fab | scFv(VL-VH) | GS_20 | Human IgG1 (knob, LALA) | Human IgG1 (hole, LALA) |
| A | PR003733 | PR001848 | PR003366 | Fab | scFv(VL-VH) | GS_20 | Human IgG1 (knob, LALA) | Human IgG1 (hole, LALA) |

(continued)

| Stru ctur e No. | Bispecific antibody molecules | CD3 antib ody | B7-H4 antibody | Structur e of CD3 end | Structure of B7-H4 end | Linker peptide | Fc type of CD3 end (mutation) | Fc type of B7-H4 end (mutation) |
|---|---|---|---|---|---|---|---|---|
| C | PR007078 | PR00 3886 | Bivalent PR007441, addition (S49C, I70C) | Fab | VH_A-VH_B (VH_A and VH_B are identical) | GS_15 | Human IgG1 (knob, LALA) | Human IgG1 (hole, LALA) |
| C | PR007168 | PR00 3886 | PR007440 and PR007441, each addition (S49C, I70C) | Fab | VH_A-VH_B (VH_A and VH_B are different) | GS_15 | Human IgG1 (knob, LALA) | Human IgG1 (hole, LALA) |

Table 4. Structure of TAA×4-1BB bispecific antibody II

| Structu re No. | Bispecific antibody molecules | 4-1BB antibody | Tumor antigen (TAA) | TAA antibod y | 4-1BB end structure | TAA end structure | Linker peptide |
|---|---|---|---|---|---|---|---|
| B | PR004281 | PR00446 9 | B7-H4 | PR0024 08 | VH | IgG | None |
| B | PR004282 | PR00446 9 | B7-H4 | PR0024 08 | VH | IgG | GS_6 |
| B | PR004359 | PR00183 8 | B7-H4 | PR0024 10 | VH | IgG | H1_15-RT |
| B | PR003338 | PR004469 | B7-H4 | PR002408 | VH | IgG | H1_15-RT |
| B | PR002828 | PR00183 8 | HER2 | PR0002 10 | VH | IgG | H1_15-RT |
| Linker (L) sequences that can be used herein in the bispecific antibody molecules in the sequence list are shown in Table 5 below. | | | | | | | |

Table 5. Sequence listing for linker peptides

| Name of linker peptide | Length of linker peptide | Sequence of linker peptide | SEQ ID NO: |
|---|---|---|---|
| GS_6 | 6 | GGSGGS | 93 |
| GS_15 | 15 | GGGGSGGGGSGGGGS | 124 |
| GS_20 | 20 | GGGGSGGGGSGGGGSGGGGS | 94 |
| H1_15-RT | 17 | EPKSSDKTHTPPPPRT | 95 |
| L-H1_15-RT | 18 | LEPKSSDKTHTPPPPRT | 96 |

The amino acid sequence numbers of the polypeptide chains of the bispecific antibody molecules of the present invention in the sequence list are shown in Table 6 below.

Table 6. Sequence listing for bispecific antibody molecules of the present invention

| Antibody No. | Polypeptide chain-1 | Polypeptide chain-2 | Polypeptide chain-3 |
|---|---|---|---|
| PR003733 | 81 | 86 | 87 |
| PR003899 | 81 | 88 | 87 |

(continued)

| Antibody No. | Polypeptide chain-1 | Polypeptide chain-2 | Polypeptide chain-3 |
|---|---|---|---|
| PR003338 | 82 | 85 | / |
| PR004281 | 82 | 89 | / |
| PR004282 | 82 | 90 | / |
| PR004359 | 83 | 91 | / |
| PR002828 | 80 | 84 | / |
| PR007078 | 113 | 114 | 115 |
| PR007168 | 113 | 114 | 116 |
| Note: "/" indicates that the polypeptide chain is absent | | | |

The CDR sequence numbers of the bispecific antibodies obtained in the present invention are shown in Table 7 below.

Table 7. Sequence listing for two antigen-binding domains corresponding to bispecific antibodies

| Bispecific antibody molecules | Antigen - binding domain number | LCDR 1 | LCDR 2 | LCDR 3 | HCDR 1 | HCDR 2 | HCDR 3 |
|---|---|---|---|---|---|---|---|
| PR003338; PR004281; PR004282 | B7-H4 | 42 | 49 | 56 | 9 | 20 | 32 |
| | 4-1BB | | | | 7 | 22 | 30 |
| PR004359 | B7-H4 | 43 | 49 | 57 | 10 | 21 | 33 |
| | 4-1BB | | | | 7 | 18 | 30 |
| PR002828 | HER2 | 40 | 47 | 54 | 6 | 17 | 29 |
| | 4-1BB | | | | 7 | 18 | 30 |
| PR003733 | B7-H4 | 43 | 49 | 57 | 10 | 21 | 33 |
| | CD3 | 41 | 48 | 55 | 8 | 19 | 31 |
| PR003899 | B7-H4 | 43 | 49 | 57 | 10 | 21 | 33 |
| | CD3 | 41 | 48 | 55 | 8 | 19 | 31 |
| PR007078 | B7-H4 | | | | 100 | 104 | 102 |
| | CD3 | 41 | 48 | 55 | 8 | 19 | 31 |
| PR007168 | B7-H4 | | | | 100 | 104 | 102 |
| | | | | | 97 | 103 | 99 |
| | CD3 | 41 | 48 | 55 | 8 | 19 | 31 |

The amino acid sequences of the CDRs of the bispecific antibodies obtained in the present invention and the corresponding SEQ ID NOs are shown in Table 8.

Table 8. Amino acid sequences of antibody CDRs and corresponding sequence numbers (Chothia)

| Heavy chain | SEQ ID NO | Amino acid sequence | Light chain | SEQ ID NO | Amino acid sequence |
|---|---|---|---|---|---|
| HCDR1 | 6 | GFNIKDT | LCDR1 | 40 | RASQDVNTAVA |
| | 7 | GFTFSNY | | 41 | RSSTGAVTTSNYAN |
| | 8 | GFTFSTY | | 42 | RASQSISSNLG |
| | 9 | GFTFRSF | | 43 | RASQSVSSNLA |
| | 10 | EDTFSSY | | | |
| | 97 | GFAFSNY | | | |
| | 100 | GFTFTDF | | | |
| HCDR3 | 29 | WGGDGFYAMDY | LCDR3 | 54 | QQHYTTPPT |
| | 30 | EGSHGTDDSHYDVDV | | 55 | ALWYSNLWV |
| | 31 | HGNFGNSYVSWFAY | | 56 | QQYQSWPPLT |
| | 32 | GGGLRWYFAY | | 57 | QQYKNWPFT |
| | 33 | GGPYFDY | | | |
| | 99 | DRFGDDYYYGMNV | | | |
| | 102 | FSTGWHRIEYFQH | | | |

The FWR sequence numbers of the bispecific antibodies obtained in the present invention are shown in Table 9 below.

Table 9. Sequence listing for FWRs corresponding to bispecific antibodies

| Heavy chain framework region | SEQ ID NO | Antibody No. | Light chain framework regions | SEQ ID NO | Antibo dy No. |
|---|---|---|---|---|---|
| HFWR1 | 1 | PR000210; PR007078; PR007168 | LFWR1 | 36 | PR000 210 |
| | 2 | PR001848; PR001838; PR004469 | | 37 | PR003 886; PR001 848 |
| | 3 | PR002408 | | 38 | PR002 408 |
| | 4 | PR003366; PR002410 | | 39 | PR003 366; PR002 410 |
| | 5 | PR003886 | | | |

(continued)

| Heavy chain framework region | SEQ ID NO | Antibody No. | Light chain framework regions | SEQ ID NO | Antibo dy No. |
|---|---|---|---|---|---|
| HFWR2 | 11 | PR000210 | LFWR2 | 44 | PR000 210 |
| | 12 | PR004469; PR001838 | | 45 | PR003 886 PR001848 |
| | 13 | PR001848 | | 46 | PR002 410; PR002 408; PR003 366 |
| | 14 | PR002408 | | | |
| | 15 | PR003366; PR002410 | | | |
| | 16 | PR003886 | | | |
| | 117 | PR007078 (bivalent PR007441); PR007168 (PR007441) | | | |
| | 120 | PR007168 (PR007440) | | | |
| HFWR3 | 23 | PR000210 | LFWR3 | 50 | PR000 210 |
| | 24 | PR004469; PR001838 | | 51 | PR003 886; PR001 848 |
| | 25 | PR001848 | | 52 | PR002 408 |
| | 26 | PR002408 | | 53 | PR003 366; PR002 410 |
| | 27 | PR003366; PR002410 | | | |
| | 28 | PR003886 | | | |
| | 118 | PR007078; PR007168 | | | |
| | 121 | PR007168 | | | |
| HFWR4 | 34 | PR001848; PR000210; PR002408; PR002410; PR003366; PR003886 | LFWR4 | 58 | PR000 210 |
| | | | | | |
| | | | | 59 | PR003 886; PR001 848 |
| | | | | 60 | PR002 408 |
| | 35 | PR004469; PR001838; PR007168 | | 61 | PR003 366; PR002 410 |
| | 119 | PR007078; PR007168 | | | |

Example 2: Binding Activity and *In-Vitro* Functional Efficacy of B7-H4×CD3

2.1. FACS assay for the binding capacity of B7-H4×CD3 bispecific antibody to B7-H4 and CD3

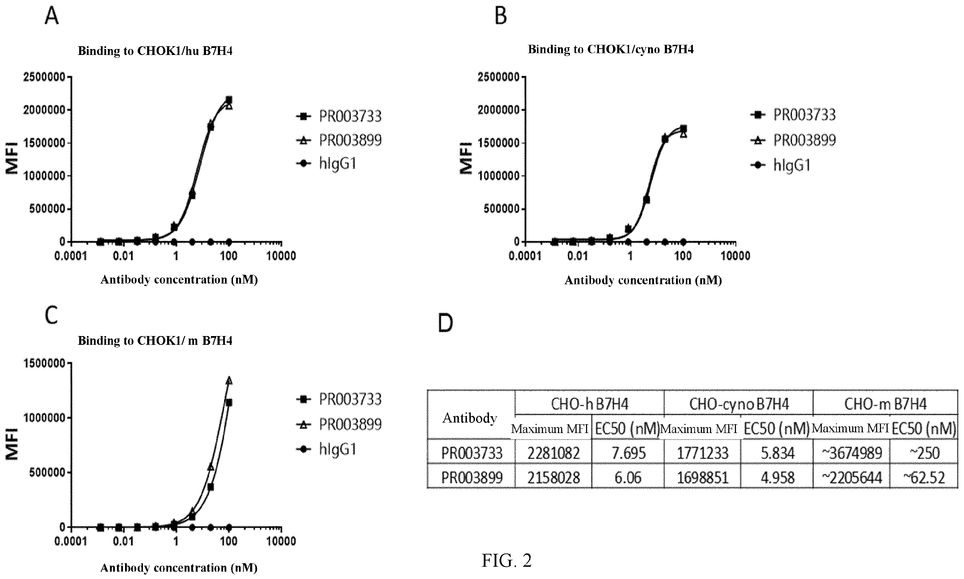

The CHO-K1 cell strain CHO-K1/huB7-H4 over-expressing human B7-H4 (produced in-house by Harbour BioMed), the CHO-K1 cell strain CHO-K1/cynoB7-H4 over-expressing cynomolgus monkey B7-H4 (produced in-house by Harbour BioMed), the CHO-K1 cell strain CHO-K1/mB7-H4 over-expressing murine B7-H4 (produced in-house by Harbour BioMed), SK-BR-3 and MDA-MB-468 cells were digested. T cells were isolated using the human T cell isolation kit (Miltenyi, #130-096-535) as described in the method of the instruction, and resuspended with PBS containing 2% FBS. The cell density was adjusted to $1 \times 10^6$ cells/mL. The cells were seeded onto a 96-well V-bottom plate (Corning, #3894) at 100 μL/well, followed by the addition of test antibodies diluted in a 3-fold gradient at a concentration that was 2 times the final concentration, each at 100 $\mu$L/well. The cells were incubated away from light at 4 °C for 2 h. Thereafter, the cells in each well were rinsed twice with 100 $\mu$L of pre-cooled PBS containing 2% BSA, and centrifuged at 500g at 4 °C for 5 min, and then the supernatant was discarded. Then each well was added with 100 $\mu$L of fluorescent secondary antibody (Alexa Fluor 488-conjugated AffiniPure Goat Anti-Human IgG, Fcy Fragment Specific, Jackson, #109-545-098, diluted in a 1:500 ratio), and the plate was incubated away from light at 4 °C for 1 h. The cells in each well were rinsed twice with 100 $\mu$L of pre-cooled PBS containing 2% FBS, and centrifuged at 500 g at 4 °C for 5 min, and then the supernatant was discarded. Finally, the cells in each well were resuspended with 200 $\mu$L of pre-cooled PBS containing 2% BSA, and the fluorescence signal values were read using an ACEA Novocyte3000 flow cytometer. The results of B7-H4$\times$CD3 bispecific antibody molecules PR003899 and PR003733 binding to CHO-K1/huB7-H4, CHO-K1/cynoB7-H4, and CHO-K1/mB7-H4 cells are shown in A-D of FIG. 2, wherein D of FIG. 2 summarizes the MFI maximums and $EC_{50}$ values of A-C FIG. 2. The results show that the binding activities of the bispecific antibody molecules PR003733 and PR003899 to B7-H4 were essentially identical, since both of the molecules used the same antiB7-H4 ScFv fragment.

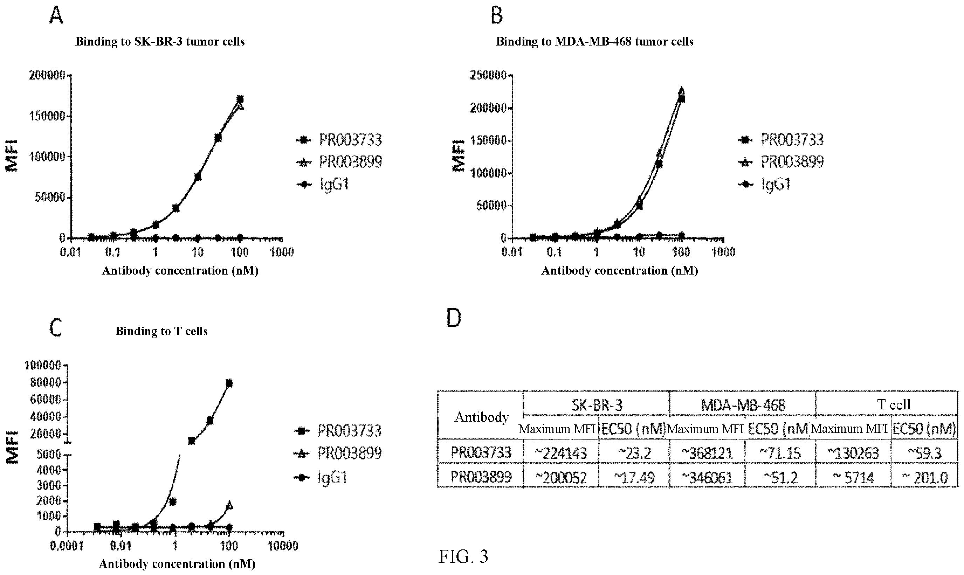

The results of B7-H4$\times$CD3 bispecific antibody molecules PR003899 and PR003733 binding to SK-BR-3 and MDA-MB-468 cells are shown in A-B of FIG. 3. The results show that the binding activities of the bispecific antibody molecules to B7-H4 on 2 types of tumor cells were essentially identical. The results of B7-H4$\times$CD3 bispecific antibody molecules binding to human T cells are shown in C of FIG. 3. The results show that the binding activity of PR003733 to T cells was significantly higher than that of PR003899. D of FIG. 3 summarizes the MFI maximums and $EC_{50}$ values of A-C FIG. 3.

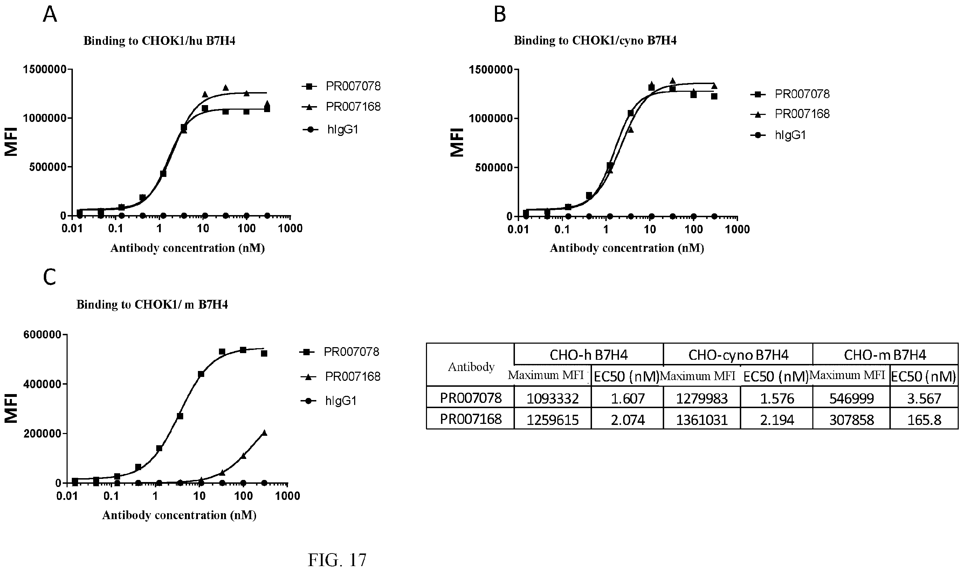

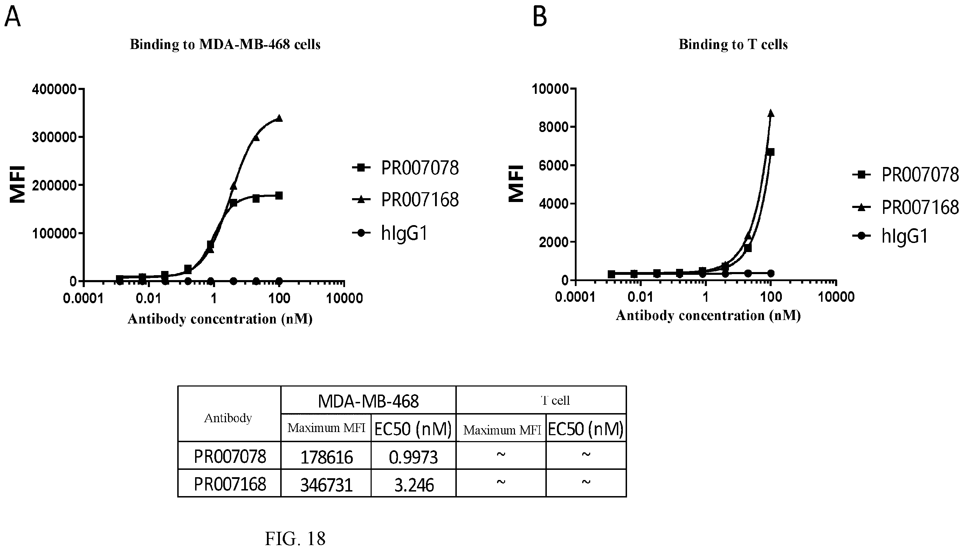

The binding activities of the bispecific antibody molecules PR007078 and PR007168 to CHOK1 cells over-expressing human and monkey B7-H4 were substantially identical (A, B, D of FIG. 17), but PR007078 bound to murine B7-H4 more strongly than PR007168 (C of FIG. 17). PR007168 bound to the tumor cells with a low level of B7-H4 expression more strongly than PR007078 (A, C of FIG. 18). Since both of the molecules used the same anti-CD3 antibody fragment, their binding capacities to CD3 were similar (B of FIG. 18).

## 2.2. T cell killing assay

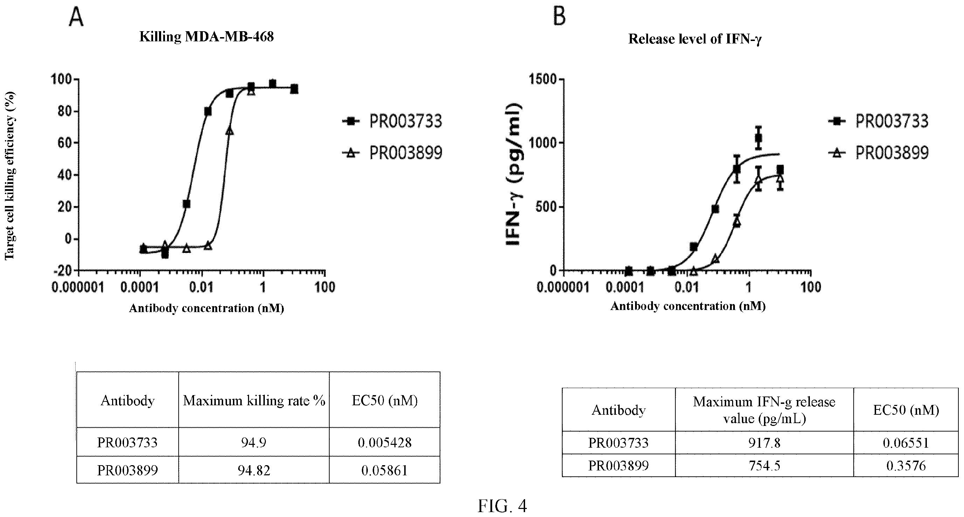

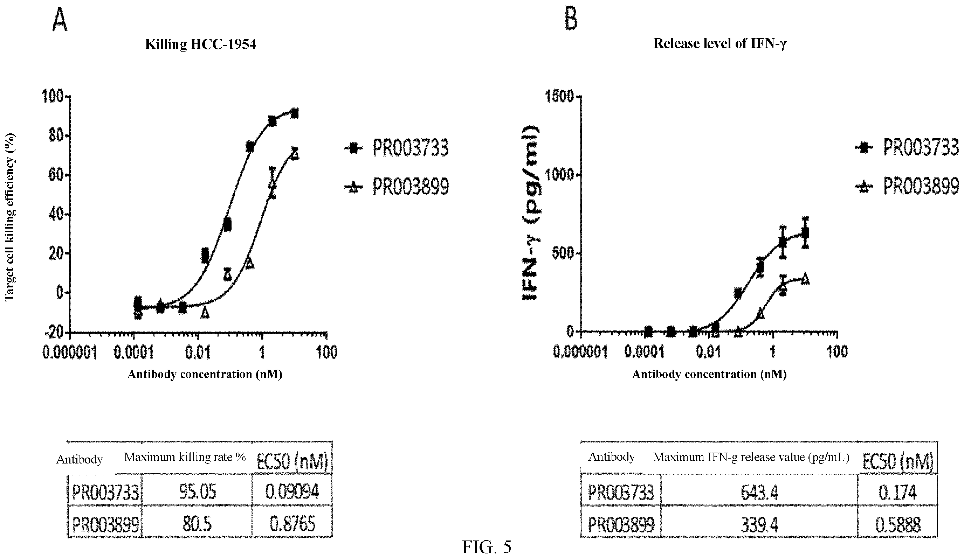

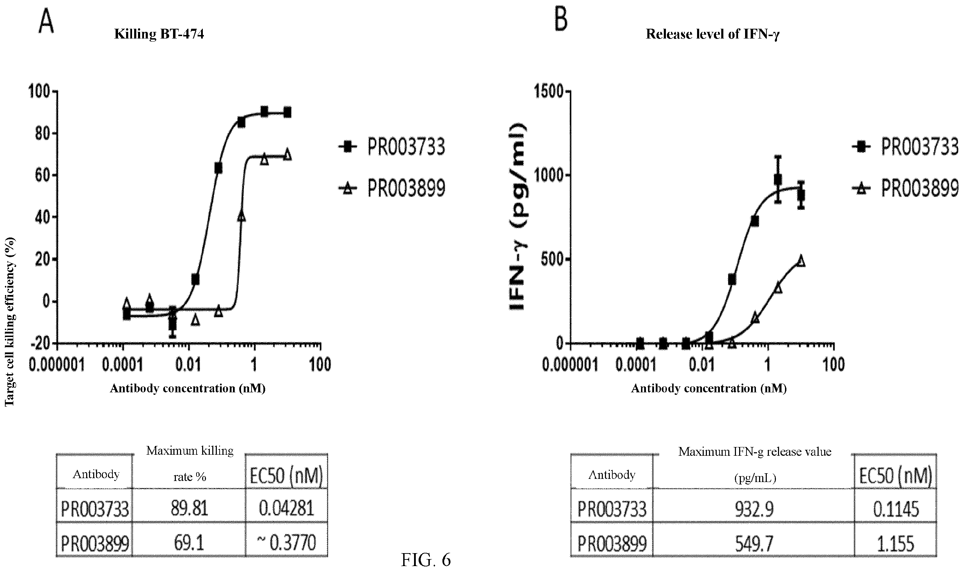

In the experiment, human primary T cells were used as effector cells, and cell lines MDA-MB-468, HCC-1954 or BT-474 that expressed different levels of B7-H4 were used as target cells. The killing efficiency was reflected by the conductivity of the target cells measured using an RTCA instrument from ACEA. A 96-well E-plate was first equilibrated with 50 $\mu$L of complete medium. Target cells were digested, resuspended in RPM1640 complete medium containing 10% fetal bovine serum and diluted to $4\times10^5$/mL. The cell suspension was plated on the 96-well E-plate at 50 $\mu$L/well, i.e., $2\times10^4$/well, and incubated overnight at 37 °C. The next day, primary T cells were isolated using the T cell isolation kit (Miltenyi, #130-096-535) according to the method of the instruction. 50 $\mu$L of fresh culture medium containing $2 \times 10^5$ T cells was added to each well, followed by the addition of 50 $\mu$L of antibodies diluted in a $4\times$ concentration gradient with a maximum final concentration of 10 nM. A total of 8 concentrations were set in duplicate for each antibody. The conductivity of the target cells was measured in real time. The value at hour 24 was used to calculate the target cell killing efficiency = (1 - sample/blank control) $\times$ 100%. The supernatant was collected after 24 hours and the concentration of IFN-$\gamma$ was detected by ELISA method. The instructions of IFN gamma Human Uncoated ELISA Kit (Thermo, #88-7316-77) were referred to for the ELISA method. The results of B7-H4$\times$CD3 bispecific antibody molecules PR003733 and PR003899 killing MDA-MB-468, HCC-1954 and BT-474 tumor cells are shown in A of FIG. 4, A of FIG. 5 and A of FIG. 6, respectively. The results show that both PR003733 and PR003899 could effectively killing tumor cells, and that PR003733 has a higher killing efficiency than PR003899. The release levels of IFN-$\gamma$ promoted by the B7-H4$\times$CD3 bispecific antibody molecules are shown in B of FIG. 4, B of FIG. 5, and B of FIG. 6, respectively.

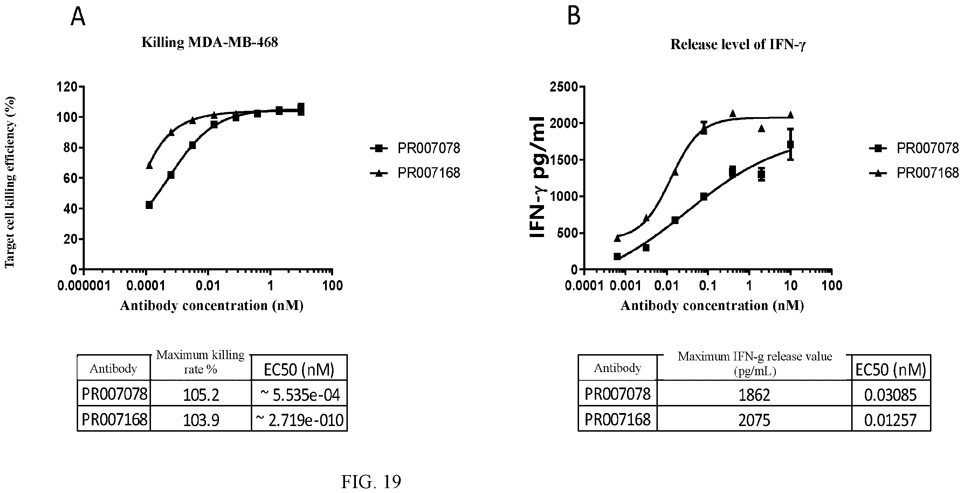

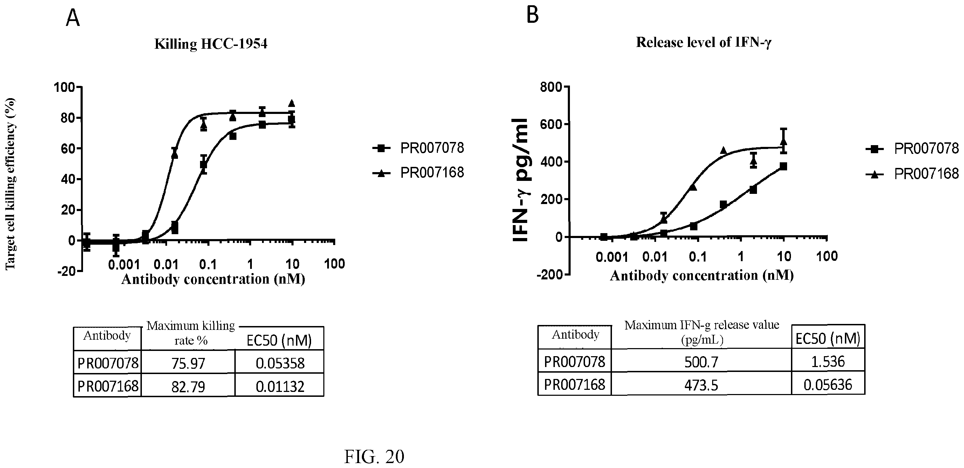

The B7-H4$\times$CD3 bispecific antibody molecules PR007078 and PR007168 could effectively kill tumor cells, and the killing efficiency of PR007168 was higher than that of PR007078 (see A of FIG. 19 and A of FIG. 20), while the cytokine IFN-$\gamma$ release level of PR007168 was higher than that of PR007078 (see B of FIG. 19 and B of FIG. 20).

Example 3: Binding Activity and *In-Vitro* Functional Efficacy of B7-H4$\times$4-1BB

3.1. FACS assay for the binding capacity of B7-H4$\times$ 4-1BB bispecific antibody to B7-H4 and 4-1BB

The CHO-K1 cell strain CHO-K1/huB7-H4 over-expressing human B7-H4 (produced in-house by Harbour BioMed), the CHO-K1 cell strain CHO-K1/cynoB7-H4 over-expressing cynomolgus monkey B7-H4 (produced in-house by Harbour BioMed), the CHO-K1 cell strain CHO-K1/mB7-H4 over-expressing murine B7-H4 (produced in-house by Harbour BioMed), SK-BR-3, the CHO-K1 cell strain CHO-K1/h4-1BB over-expressing human 4-1BB (produced in-house by Harbour BioMed), and the CHO-K1 cell strain CHO-K1/cyno4-1BB over-expressing cynomolgus monkey 4-1BB (produced in-house by Harbour BioMed) were digested, and they were then resuspended with PBS containing 2% FBS. The cell density was adjusted to $1\times10^6$ cells/mL. The cells were seeded onto a 96-well V-bottom plate (Corning, #3894)

at 100 μL/well, followed by the addition of test antibodies diluted in a 3-fold gradient at a concentration that was 2 times the final concentration, each at 100 μL/well. The cells were incubated away from light at 4 °C for 2 h. Thereafter, the cells in each well were rinsed twice with 100 μL of pre-cooled PBS containing 2% BSA, and centrifuged at 500g at 4 °C for 5 min, and then the supernatant was discarded. Then each well was added with 100 μL of fluorescent secondary antibody (Alexa Fluor 488-conjugated AffiniPure Goat Anti-Human IgG, Fcy Fragment Specific, Jackson, #109-545-098, diluted in a 1:500 ratio), and the plate was incubated away from light at 4 °C for 1 h. The cells in each well were rinsed twice with 100 μL of pre-cooled PBS containing 2% FBS, and centrifuged at 500 g at 4 °C for 5 min, and then the supernatant was discarded. Finally, the cells in each well were resuspended with 200 μL of pre-cooled PBS containing 2% BSA, and the fluorescence signal values were read using an ACEA Novocyte3000 flow cytometer.

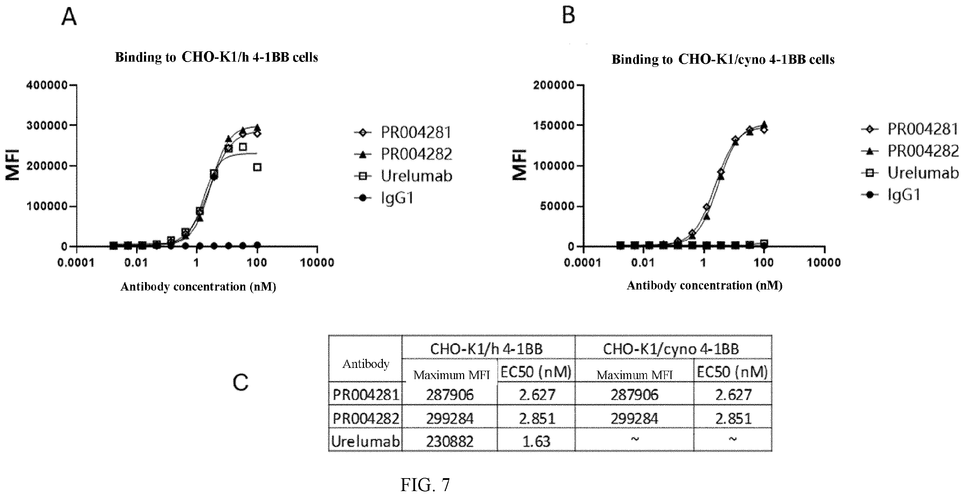

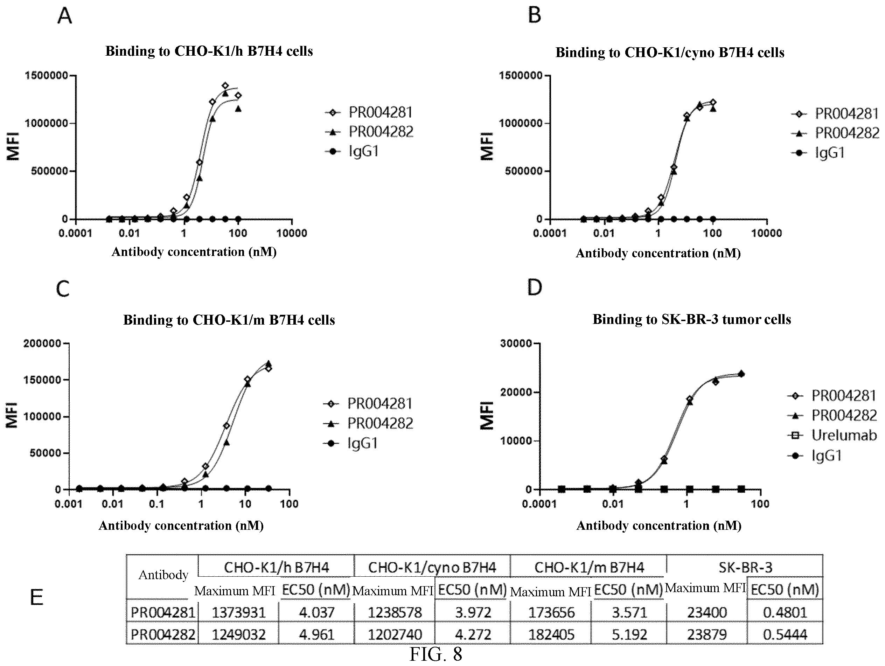

The results of B7-H4×4-1BB bispecific antibody molecules PR004281 and PR004282 binding to CHO-K1/hu4-1BB and CHO-K1/cyno4-1BB are shown in A-C of FIG. 7, wherein C of FIG. 7 summarizes the MFI maximums and $EC_{50}$ values of A-B of FIG. 7. The results show that the binding activities of PR004281 and PR004282 to human and monkey 4-1BB were similar, while Urelumab did not bind to monkey 4-1BB, as reported in the literature. The results of the B7-H4×4-1BB bispecific antibody molecules binding to CHO-K1/huB7-H4, CHO-K1/cynoB7-H4, CHO-K1/mB7-H4 and SK-BR-3 cells are shown in A-E of FIG. 8, wherein E of FIG. 8 summarizes the MFI maximums and $EC_{50}$ values of A-D of FIG. 8. The results show that PR004281 and PR004282 had similar binding activity to human, monkey, murine 4-1BB and tumor cells SK-BR-3.

3.2. T-cell activation assay

1 mg/mL OKT3 (eBiosciences, #16-0037-85) was diluted with PBS. A 96-well plate (Corning, #3599) was coated with 10 μg/mL OKT3 at 100 μL/well, and incubated at 4 °C overnight. The next day, SK-BR-3, JIMT-1 or CHO-K1/CD32b cells were digested and resuspended in a complete medium, and the cell density was adjusted to $4×10^5$ cell/mL for later use. Human CD3-positive T cells were isolated from human PBMCs using a MACS kit (Miltenyi Biotec, #130-096-535). The previous day's OKT3 for coating was washed off the 96-well plate, and 50 μL of purified T cells were added at $1×10^5$ cell/well. 50 μL of SK-BR-3, JIMT-1 or CHO-K1/CD32b cells were then added to the 96-well plate at $2×10^4$ cell/well. Then 50 μL of the B7-H4×4-1BB bispecific antibody or the control monoclonal antibody at a corresponding concentration was added, and the plate was incubated in an incubator at 37 °C. After 48 hours, 80 μL of the supernatant was collected and assayed for IL-2 content using an ELISA kit (Invitrogen, #88-7025-88). An assay was carried out according to the instructions of the manufacturer.

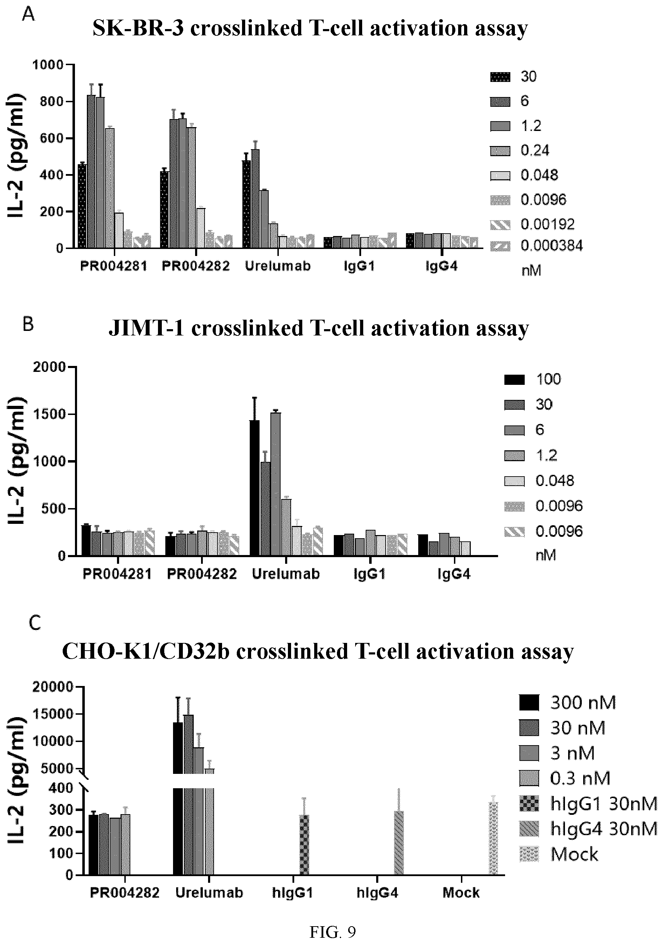

A of FIG. 9 shows that B7-H4×4-1BB bispecific antibody molecules PR004281 and PR004282 had strong effects of activating T cells to secrete IL-2 under the crosslinking of B7-H4 on the surface of SK-BR-3 cells. JIMT-1 cells were B7-H4 negative cells, and could not crosslink B7-H4×4-1BB bispecific antibody molecules, so PR004281 and PR004282 did not have the function of activating T cells in the presence of JIMT-1 (B of FIG. 9). Meanwhile, the LALA mutation on the bispecific antibody molecules reduced the binding of the bispecific antibody to Fc receptor CD32b, and thus the bispecific antibody did not have the function of activating T cells in the presence of CHO-K1/CD32b cells (C of FIG. 9). It suggests that B7-H4×4-1BB could only play a role in activating T cells in a tumor environment with B7-H4 high-expression, so that the peripheral effect was reduced, and further, the peripheral toxicity was reduced. The control molecule urelumab functioned independently of crosslinking (A-B of FIG. 9), and the activity thereof was further enhanced in the presence of CD32b crosslinking (C of FIG. 9).

Example 4: Ratio of T Cells to Tumor Cells in Human Breast Cancer Tissue

The pathological tissue chip was the BRC1021 breast cancer tissue chip purchased from Guilin Fanpu Biotech, Inc. Paraffin sections had a thickness of 4 μm and were taken with positive control tissue. Dewaxing and washing were performed; antigen retrieval was performed as follows: citric acid at pH 6 was added, the mixture was heated at 125 °C for 5 minutes, sealed for 10 minutes, and cooled at room temperature for 30 minutes; the mixture was washed with water and then with 0.3% hydrogen peroxide for 5 minutes, and then washed with TBST for 3 times for 5 minutes; Dako blocking buffer was used directly, and blocked in an incubation box at room temperature for 20 minutes; the blocking buffer was removed, the rabbit anti-human CD3 primary antibody was added, the antibody diluent Dako was used directly, the mixture was incubated for 60 minutes in an incubation box at room temperature, and a control was substituted with Rabbit IgG; the mixture was washed with TBST for three times, five minutes each time; a secondary antibody, Anti-Rabbit (EnVision + System-HRP Labelled Polymer) was incubated in the incubation box for 30 minutes at room temperature; the mixture was washed with TBST for three times, five minutes each time; after DAB color development, 0.85 mL of distilled water was added into reagents according to the sequence of the reagents A to B to C in an amount of 50 μL, and the mixture was incubated for 5 minutes in an incubation box at room temperature, then washed with distilled water, and counterstained with hematoxylin. The chip was observed under a microscope followed by sealing and reading.

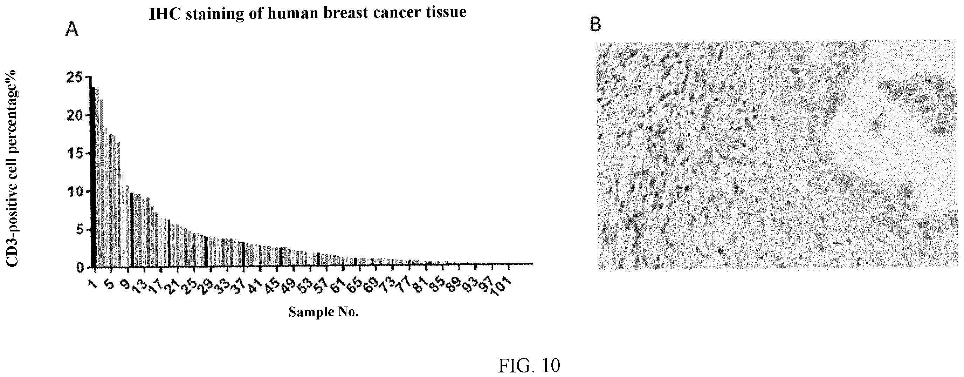

The results (A of FIG. 10) show that the proportion of T cells in the tumor tissue was low, and the proportion of T cells in most samples was lower than 10%, indicating that under pathological conditions, the proportion of T cells to tumor tissue was much lower than that of an *in-vitro* experiments. B of FIG. 10 shows one of the sample examples.

Example 5: *In-Vitro* Killing Assay for B7-H4×CD3 At Low Ratio of T Cells to Tumor Cells

The method of this example was analogous to that of Example 2.2, using MDA-MB-468 tumor cells as target cells. The killing efficiency was reflected by the conductivity of the target cells measured using an RTCA instrument from ACEA. A 96-well E-plate was first equilibrated with 50 μL of complete medium. Target cells were digested, resuspended in RPM1640 complete medium containing 10% fetal bovine serum and diluted to $4\times10^5$/mL. The cell suspension was plated on the 96-well E-plate at 50 μL/well, i.e., $2\times10^4$/well, and incubated overnight at 37 °C. The next day, primary T cells were isolated using the T cell isolation kit (Miltenyi, #130-096-535) according to the method of the instruction. 50 μL of fresh T cells at different densities were added to each well at a ratio of T cells to tumor cells of 0.03:1 to 10:1, followed by addition of 50 μL of antibody at a final concentration of 2 nM. This concentration was the saturation concentration in the killing experiment with a ratio of effector cells to target cells (E:T) of 10:1 (A of FIG. 4). The conductivity of the target cells was measured in real time. The values at 24 h, 48 h, 72 h and 96 h were used to calculate the target cell killing efficiency = (1 - sample/blank control) × 100%.

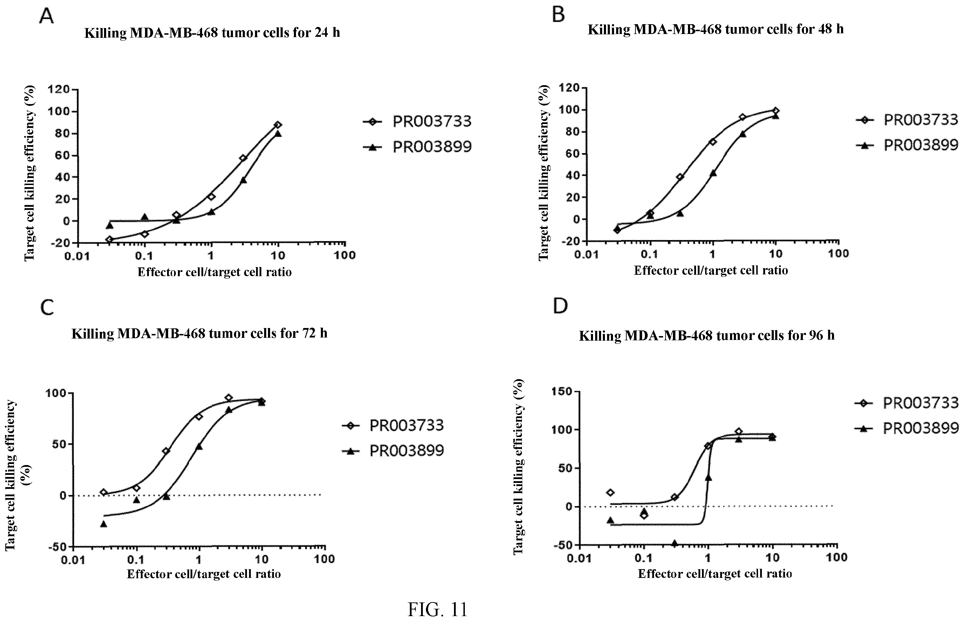

The results of B7-H4×CD3 bispecific antibody molecules PR003733 and PR003899 killing MDA-MB-468 tumor cells are shown in A-D of FIG. 11 (24 h to 96 h). The results show that the tumor cell efficiencies of PR003733 and PR003899 decreased with the decreased ratio of effector cells to target cells, and the antibody had basically no killing activity at a ratio below 1:3, with a consistent trend from 24 h to 96 h, indicating that the B7-H4×CD3 bispecific antibody molecules mediated a low tumor killing efficiency at a low ratio of effector cells to target cells (e.g., in a tumor environment).

Example 6: Indexes of T-Cell Depletion by Combination Use of B7-H4×CD3 and B7-H4×4-1BB

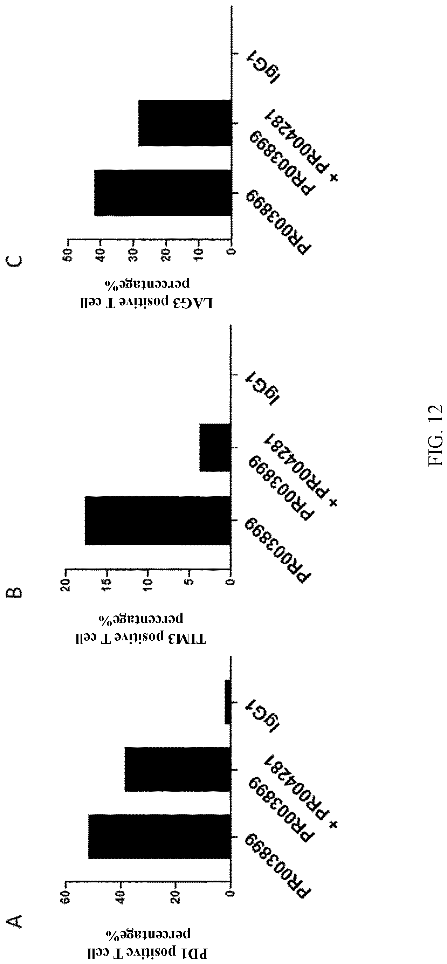

MDA-MB-468 tumor cells were used as target cells, and B7-H4 molecules were endogenously and highly expressed on the surface of the MDA-MB-468 cells. A 96-well plate was first equilibrated with 50 μL of complete medium. Target cells were digested, resuspended in RPM1640 complete medium containing 10% fetal bovine serum and diluted to $4\times10^5$/mL. The cell suspension was plated on the 96-well E-plate at 50 μL/well, i.e., $2\times10^4$/well, and incubated overnight at 37 °C. The next day, primary T cells were isolated using the T cell isolation kit (Miltenyi, #130-096-535) according to the method of the instruction. 50 μL of fresh 6666 T cells were added to each well, i.e., the ratio of effector cells to target cells (E:T) was 1:3. Antibody PR003899 alone or in combination with PR004281 was then added, with a final concentration of 1 nM. The conductivity of the target cells was detected in real time. T cells were collected at 48 h, and the expression of immune checkpoints PD1, Tim3 and Lag3 on the T cells were detected using flow cytometry, wherein the high expression of the 3 markers was usually marked as T-cell depletion. The method was as follows: the suspension cells were harvested, washed once with PBS, and resuspended in 100 μL of FACS buffer (PBS containing 2% FBS), followed by addition of 1 μL FITC anti-human CD3, 1 μL APC anti-humanPD1, 1 μL Brilliant Violet421 anti-Tim3, and 1 μL Brilliant Violet421 anti-Lag3 antibodies, and the mixture was incubated at 4 °C for 45 minutes, washed 2 times, and tested using the machine. The results show that the expression of PD1, Tim3 and Lag3, under the combination use of B7-H4×CD3 bispecific antibody PR003899 and B7-H4×4-1BB bispecific antibody PR004281, was reduced to a certain extent, and especially the expression of Tim3 was significantly reduced compared with that of B7-H4×CD3 used alone after 48 h of incubation (A-C of FIG. 12).

Example 7: Combination Use of B7-H4×CD3 and B7-H4×4-1BB Capable of Significantly Promoting Tumor Killing in Later Stage at Low Ratio of T Cells to Tumor Cells

The method of this example was analogous to that of Example 6, and the killing rate of target cells at different time points was examined. MDA-MB-468 tumor cells were used as target cells. A 96-well plate was first equilibrated with 50 μL of complete medium. Target cells were digested, resuspended in RPM1640 complete medium containing 10% fetal bovine serum and diluted to $4\times10^5$/mL. The cell suspension was plated on the 96-well E-plate at 50 μL/well, i.e., $2\times10^4$/well, and incubated overnight at 37 °C. The next day, primary T cells were isolated using the T cell isolation kit (Miltenyi, #130-096-535) according to the method of the instruction. 50 μL of fresh 6666 T cells were added to each well, i.e., the ratio of effector cells to target cells (E:T) was 1:3. Antibody PR003899 alone or in combination with PR004281 was then added, with a final concentration of 1 nM. The conductivity of the target cells was detected in real time.

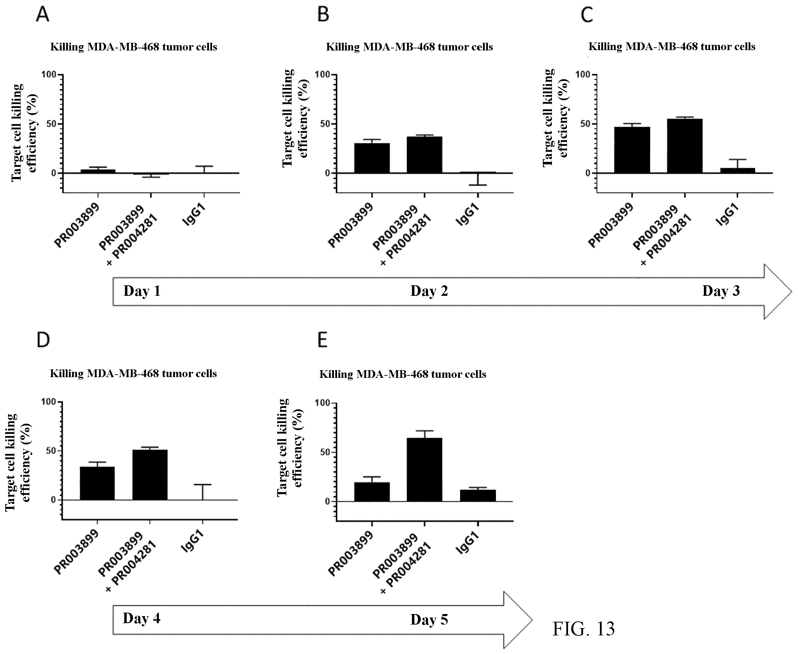

The results of B7-H4×CD3 bispecific antibody molecule PR003899 killing MDA-MB-468 tumor cells are shown in A-E of FIG. 13 (day 1 to day 5). The results show that the killing efficiency of PR003899 alone peaked at day 3, but slowly decreased after day 4, probably due to T-cell depletion. The killing efficiency of PR003899 and PR004281 used together was not significantly different from that of PR003899 used alone in the first 3 days, but the killing efficiency remained increasing in the later period of day 4 to day 5, with an increasing difference from PR003899 alone. It was likely due to the reduced T-cell depletion and apoptosis by the combination use of PR003899 and PR004281.

Example 8: Combination Use of B7-H4×CD3 and B7-H4×4-1BB in Increasing T-Cell Division and Proliferation While Enhancing Killing Activity

The method of this example was analogous to that of Example 5. SKBR3 tumor cells endogenously and highly expressing B7-H4 molecules were used as target cells. A 96-well plate was first equilibrated with 50 μL of complete medium. Target cells were digested, resuspended in RPM1640 complete medium containing 10% fetal bovine serum and diluted to $4\times10^5$/mL. The cell suspension was plated on the 96-well E-plate at 50 μL/well, i.e., $2\times10^4$/well, and incubated overnight at 37 °C. The next day, primary T cells were isolated using the T cell isolation kit (Miltenyi, #130-096-535) according to the method of the instruction. 50 μL of labeled T cells were added to each well at a ratio of effector cells to target cells (E:T) of 1:5. The antibodies PR007078 and PR007168 alone or in combination with PR004282 were then added. The conductivity of the target cells was detected in real time. And the target cell killing efficiency was calculated on day 7.

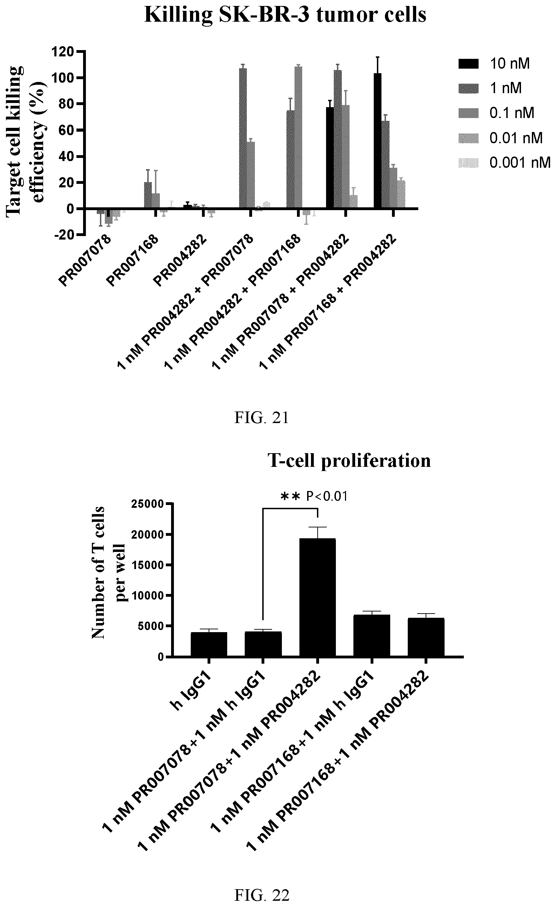

The results show (FIG. 21) that, in the case of combination use of B7-H4×CD3 bispecific antibodies PR007078 and PR007168 and B7-H4×4-1BB bispecific antibody PR004282, after 7 days of incubation, PR007078 alone had no killing effect at a low ratio of effector cells to target cells (E:T), and PR007078 and B7-H4×4-1BB bispecific antibody PR004282 used together had a strong killing effect. PR007168 itself had a certain killing effect, and PR007168 and PR004282 used together had an enhanced killing effect.

In another experiment, 1 μM CSFE-labeled T cells were co-cultured with SKBR3 target cells, and the number of living T cells and passage numbers of T cells undergoing division were counted using flow cytometer on day 7, and Annexin V staining was used for detecting apoptotic T cells.

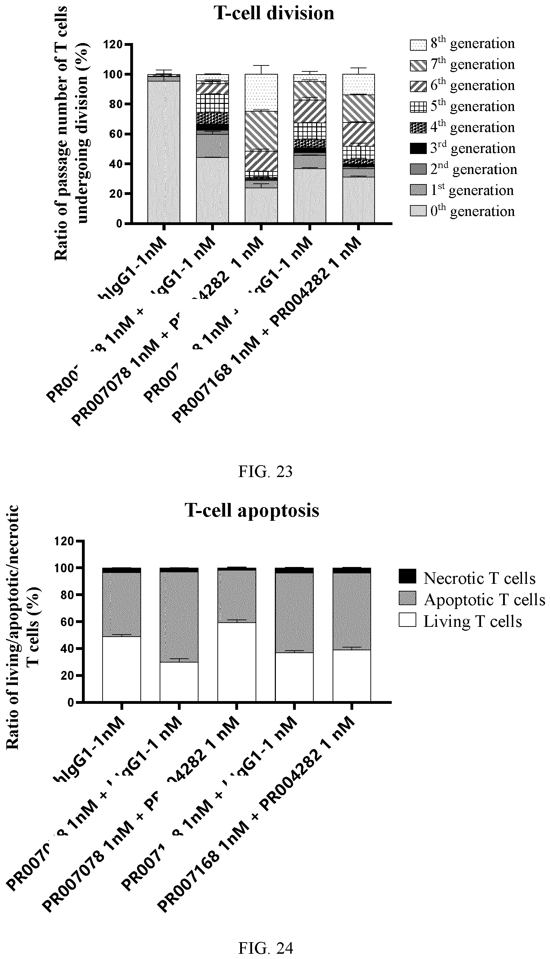

The results show that the combination use of PR007078 and PR004282 significantly promoted the absolute number of T cells (FIG. 22) and significantly increased the passage numbers of T cells undergoing division (FIG. 23), and meanwhile, significantly reduced the rate of apoptotic T cells (FIG. 24), compared to use of an antibody alone. PR007168 alone had a strong effect in the experiment, and PR007168 and PR004282 used together had no significant effect on T cells themselves.

Example 9: *In-Vitro* Killing Assay for Different BsAb Combinations

9.1. Determination for PR002408 and PR002410 binding to B7-H4 epitopes

The present invention proves that the method for binding PR002408 and PR002410 to different epitopes of B7-H4 comprises the following steps: using the ForteBio Octet Red96e platform. In the first step, 100% signal of an antibody was obtained as follows: the final signal for the primary antibody (PR002408) binding to B7-H4 was recorded as the 100% signal of the antibody. In the second step, after the capture of the primary antibody, the SA sensor was immersed in a mixture of the primary antibody and the secondary antibody (PR002410), and the difference in signals after immersion of the sensor in the antibody mixture was recorded as the signal of the secondary antibody. The inhibition rate was calculated according to the following formula:

$$\text{Inhibition (\%)} = (A - B)/A \times 100$$

A: 100% signal of an antibody (obtained from the first step), B: the signal of the antibody as the secondary antibody (obtained from the second step).

If the inhibition rate obtained was greater than 85 (%), it means that the epitopes of the two antibodies were completely overlapped; if the inhibition rate was less than 85 (%), it means that the epitopes to which the two antibodies bind were not completely overlapped. The results are shown in Table 10 in which there was no epitope competition between PR002408 and PR002410, indicating they bound to different antigenic epitopes of B7-H4. The results of the epitope competition experiment are shown in Table 10 below.

Table 10. Epitope competition experiment for PR002408 and PR002410

| Inhibition rate (%) | | Secondary antibody | |
|---|---|---|---|
| | | PR002408 | PR002410 |
| Primary antibody | PR002408 | 94.91 | 7.23 |
| | PR002410 | 7.12 | 94.77 |

*9.2. In-vitro killing effect of different BsAb combinations*

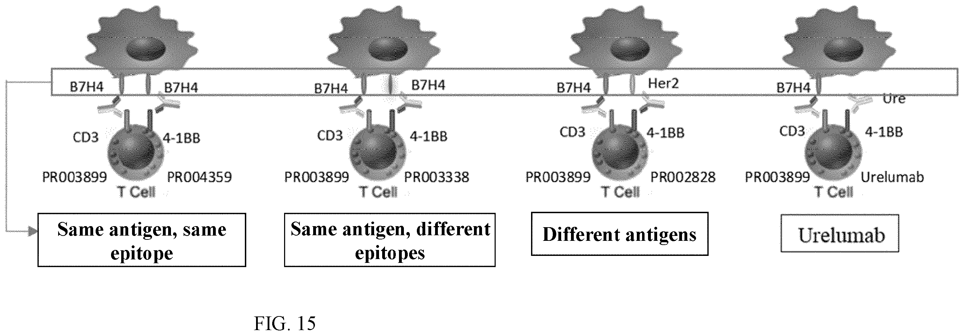

To explore the role of different binding epitopes of different TAAs or the same TAA in mediating the combination use of TAA×CD3 and TAA×4-1BB, different combinations of TAA×CD3 and TAA×4-1BB were used in this example. TAA×CD3 represented PR003899, and its TAA was anti-B7-H4; TAA×4-1BB represented PR004359, PR003338 and PR002828, while urelumab was a positive control. The TAA of PR004359 was anti-B7-H4, identical to that of PR003899; the TAA of PR003338 was anti-B7-H4 with an antigenic epitope of B7-H4 that the TAA bound to which was different from that for PR003 899; the TAA of PR002828 was anti-Her2. The schematic diagram is shown in FIG. 15.

The killing method of this example was analogous to that of Example 7, and the target cell killing rate on day 8 was measured. SK-BR-3 tumor cells were used as target cells, highly expressing both of B7-H4 and Her2. A 96-well plate was first equilibrated with 50 μL of complete medium. Target cells were digested, resuspended in RPM1640 complete medium containing 10% fetal bovine serum and diluted to $4\times10^5$/mL. The cell suspension was plated on the 96-well E-plate at 50 μL/well, i.e., $2\times10^4$/well, and incubated overnight at 37 °C. The next day, primary T cells were isolated using the T cell isolation kit (Miltenyi, #130-096-535) according to the method of the instruction. 50 μL of fresh 2000 T cells were added to each well, i.e., the ratio of effector cells to target cells (E:T) was 1:10. Antibody PR003899 alone or in combination with other TAA×4-1BB was then added, with a final PR003899 concentration of 0.5 nM and a TAA×4-1BB concentration of 2 nM or 0.2 nM. The conductivity of the target cells was detected in real time. And T cells were collected on day 8, and living T cells were stained and counted.

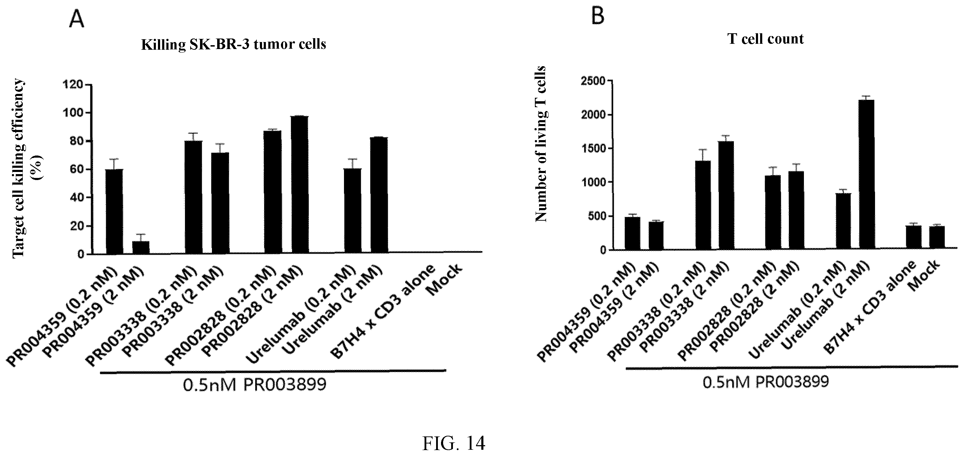

As shown in FIG. 14, PR003899 alone showed no killing effect at a low ratio of effector cells to target cells (E:T) of 1:10, while PR003899 and TAA×4-1BB used together showed a strong killing effect, and meanwhile, the amount of living T cells significantly increased, the killing rate of SK-BR-3 tumor cells was shown in A of FIG. 14, and the number of living T cells was shown in B of FIG. 14. The B7-H4 antigen-binding epitope of PR004359 was identical to that of PR003899, having an epitope competition relationship, so the synergistic effect thereof was reduced at a high concentration of 2 nM; while PR003899 had a strong synergistic effect with PR003338 of different epitopes of the same TAA or PR002828 of different TAAs. The results show that the combination use of TAA×CD3 and TAA×4-1BB bispecific antibodies of different epitopes of the same TAA or of different TAAs showed a strong synergistic killing effect, providing a potential clinical application value.

Although specific embodiments of the present invention have been described above, it will be appreciated by those skilled in the art that these embodiments are merely illustrative and that many changes or modifications can be made to these embodiments without departing from the principles and spirit of the present invention. The scope of protection of the present invention is therefore defined by the appended claims.

**Claims**

1. A bispecific antibody (BsAb) combination, comprising a bispecific antibody I as a first therapeutic agent and a bispecific antibody II as a second therapeutic agent, wherein the bispecific antibody I comprises a CD3-targeting domain and a tumor-associated antigen-targeting (TAA) domain, and the bispecific antibody II comprises a 4-1BB-targeting domain and a TAA-targeting domain; preferably, in the bispecific antibody I, the TAA-targeting domain is a B7-H4-targeting domain; and/or, in the bispecific antibody II, the TAA-targeting domain is a B7-H4-targeting domain or a Her2-targeting domain; more preferably, the B7-H4-targeting domains in the bispecific antibody I and the bispecific antibody II target different epitopes of B7-H4, respectively.

2. The BsAb combination according to claim 1, wherein the bispecific antibody I is a Fab-Fc-scFv or Fab-Fc-$(VH)_n$ asymmetric structure, and the bispecific antibody II is an IgG-VH symmetric structure, wherein n is a natural number greater than or equal to 1;

preferably, in the bispecific antibody I, the B7-H4-targeting domain is a scFv or VH_A-VH_B, wherein the VH_A and the VH_B are identical or different, the CD3-targeting domain is a Fab, and the Fab and the scFv or the VH_A-VH_B are linked to an Fc via a hinge region or a linker peptide; the Fc is preferably an Fc of an IgG1, the amino acid sequence of the linker peptide is set forth in SEQ ID NOs: 93-96, 124, and more preferably, the Fc comprises an addition, deletion or mutation of 1-3 amino acids, such as mutations L234A and L235A, "knob" mutation T366W and "Hole" mutations T366S, L368A and Y407V, and/or, "knob" mutation S354C and "Hole" mutation Y349C; and/or,

in the bispecific antibody II, the B7-H4-targeting domain or the Her2-targeting domain is an IgG, and an Fc of the IgG preferably comprises an addition, deletion or mutation of 1-3 amino acids, such as mutations L234A and L235A; the 4-1BB-targeting domain is a VH or a scFv; the B7-H4 targeting domain or the Her2-targeting domain is linked to the 4-1BB-targeting domain via a linker peptide, and the amino acid sequence of the linker peptide is preferably set forth in SEQ ID NOs: 93-96.

3. The BsAb combination according to claim 1 or 2, wherein in the bispecific antibody I, the B7-H4-targeting domain comprises a heavy chain variable region having amino acid sequences of HCDR1-3 set forth in SEQ ID NOs: 10, 21 and 33, respectively, and a light chain variable region having amino acid sequences of LCDR1-3 set forth in SEQ ID NOs: 43, 49 and 57, respectively; or, the B7-H4-targeting domain comprises VH_A-VH_B, wherein amino acid sequences of HCDR1-3 of the VH_A and the VH_B are set forth in SEQ ID NOs: 100, 104 and 102, respectively, or, amino acid sequences of HCDR1-3 of the VH_A are set forth in SEQ ID NOs: 100, 104 and 102, respectively, and amino acid sequences of HCDR1-3 of the VH_B are set forth in SEQ ID NOs: 97, 103 and 99, respectively;

the CD3-targeting domain comprises a heavy chain variable region having the amino acid sequences of HCDR1-3 set forth in SEQ ID NOs: 8, 19 and 31, respectively, and a light chain variable region having the amino acid sequences of LCDR1-3 set forth in SEQ ID NOs: 41, 48 and 55, respectively; and/or,
in the bispecific antibody II, the B7-H4-targeting domain comprises a heavy chain variable region having the amino acid sequences of HCDR1-3 set forth in SEQ ID NOs: 9, 20 and 32, respectively, and a light chain variable region having the amino acid sequences of LCDR1-3 set forth in SEQ ID NOs: 42, 49 and 56, respectively; or, the heavy chain variable region has the amino acid sequences of HCDR1-3 set forth in SEQ ID NOs: 10, 21 and 33, respectively, and the light chain variable region has the amino acid sequences of LCDR1-3 set forth in SEQ ID NOs: 43, 49 and 57, respectively; the Her2-targeting domain comprises a heavy chain variable region having the amino acid sequences of HCDR1-3 set forth in SEQ ID NOs: 6, 17 and 29, respectively, and a light chain variable region having the amino acid sequences of LCDR1-3 set forth in SEQ ID NOs: 40, 47 and 54, respectively;
the 4-1BB-targeting domain is a VH having the amino acid sequences of HCDR1-3 set forth in SEQ ID NOs: 7, 18 and 30 or SEQ ID NOs: 7, 22 and 30, respectively;
preferably, the bispecific antibody II is selected from the group consisting of:

a) the bispecific antibody II comprises the B7-H4-targeting domain and the 4-1BB-targeting domain; wherein

in the B7-H4-targeting domain, the heavy chain variable region has the amino acid sequences of HCDR1-3 set forth in SEQ ID NOs: 9, 20 and 32, respectively, and the light chain variable region has the amino acid sequences of LCDR1-3 set forth in SEQ ID NOs: 42, 49 and 56, respectively; in the 4-1BB-targeting domain, the amino acid sequences of HCDR1-3 are set forth in SEQ ID NOs: 7, 22 and 30; or,
in the B7-H4-targeting domain, the heavy chain variable region has the amino acid sequences of HCDR1-3 set forth in SEQ ID NOs: 10, 21 and 33, respectively, and the light chain variable region has the amino acid sequences of LCDR1-3 set forth in SEQ ID NOs: 43, 49 and 57, respectively; in the 4-1BB-targeting domain, the amino acid sequences of HCDR1-3 are set forth in SEQ ID NOs: 7, 18 and 30; and

b) the bispecific antibody II comprises the Her2-targeting domain and the 4-1BB-targeting domain; wherein in the Her2-targeting domain, the heavy chain variable region has the amino acid sequences of HCDR1-3 set forth in SEQ ID NOs: 6, 17 and 29, respectively, and the light chain variable region has the amino acid sequences of LCDR1-3 set forth in SEQ ID NOs: 40, 47 and 54, respectively; in the 4-1BB-targeting domain, the amino acid sequences of HCDR1-3 are set forth in SEQ ID NOs: 7, 18 and 30, respectively;

more preferably, the BsAb combination is selected from:

i) in the bispecific antibody I, the B7-H4-targeting domain comprises the heavy chain variable region having the amino acid sequences of HCDR1-3 set forth in SEQ ID NOs: 10, 21 and 33, respectively, and the light chain variable region having the amino acid sequences of LCDR1-3 set forth in SEQ ID NOs: 43, 49 and 57, respectively; the CD3-targeting domain comprises the heavy chain variable region having the amino acid sequences of HCDR1-3 set forth in SEQ ID NOs: 8, 19 and 31, respectively, and the light chain variable region having the amino acid sequences of LCDR1-3 set forth in SEQ ID NOs: 41, 48 and 55, respectively; in the bispecific antibody II, the B7-H4-targeting domain comprises the heavy chain variable region having the amino acid sequences of HCDR1-3 set forth in SEQ ID NOs: 9, 20 and 32, respectively, and the light chain variable region having the amino acid sequences of LCDR1-3 set forth in SEQ ID NOs: 42, 49 and 56, respectively; the 4-1BB-targeting domain comprises the amino acid sequences of HCDR1-3 set forth in SEQ ID NOs: 7, 22 and 30, respectively;

ii) in the bispecific antibody I, the B7-H4-targeting domain comprises the heavy chain variable region having the amino acid sequences of HCDR1-3 set forth in SEQ ID NOs: 10, 21 and 33, respectively, and the light chain variable region having the amino acid sequences of LCDR1-3 set forth in SEQ ID NOs: 43, 49 and 57, respectively; the CD3-targeting domain comprises the heavy chain variable region having the amino acid sequences of HCDR1-3 set forth in SEQ ID NOs: 8, 19 and 31, respectively, and the light chain variable region having the amino acid sequences of LCDR1-3 set forth in SEQ ID NOs: 41, 48 and 55, respectively; in the bispecific antibody II, the Her2-targeting domain comprises the heavy chain variable region having the amino acid sequences of HCDR1-3 set forth in SEQ ID NOs: 6, 17 and 29, respectively, and the light chain variable region having the amino acid sequences of LCDR1-3 set forth in SEQ ID NOs: 40, 47 and 54, respectively; the 4-1BB-targeting domain comprises the amino acid sequences of HCDR1-3 set forth in SEQ ID NOs: 7, 18 and 30, respectively;

iii) in the bispecific antibody I, the B7-H4-targeting domain comprises the VH_A and the VH_B having the amino acid sequences of HCDR1-3 set forth in SEQ ID NOs: 100, 104 and 102, respectively, and the CD3-targeting domain comprises the heavy chain variable region having the amino acid sequences of HCDR1-3 set forth in SEQ ID NOs: 8, 19 and 31, respectively, and the light chain variable region having the amino acid sequences of LCDR1-3 set forth in SEQ ID NOs: 41, 48 and 55, respectively; in the bispecific antibody II, the B7-H4-targeting domain comprises the heavy chain variable region having the amino acid sequences of HCDR1-3 set forth in SEQ ID NOs: 9, 20 and 32, respectively, and the light chain variable region having the amino acid sequences of LCDR1-3 set forth in SEQ ID NOs: 42, 49 and 56, respectively; the 4-1BB-targeting domain comprises the amino acid sequences of HCDR1-3 set forth in SEQ ID NOs: 7, 22 and 30, respectively;

iv) in the bispecific antibody I, the B7-H4-targeting domain comprises the VH_A and the VH_B having the amino acid sequences of HCDR1-3 set forth in SEQ ID NOs: 100, 104 and 102, respectively, and the CD3-targeting domain comprises the heavy chain variable region having the amino acid sequences of HCDR1-3 set forth in SEQ ID NOs: 8, 19 and 31, respectively, and the light chain variable region having the amino acid sequences of LCDR1-3 set forth in SEQ ID NOs: 41, 48 and 55, respectively; in the bispecific antibody II, the Her2-targeting domain comprises the heavy chain variable region having the amino acid sequences of HCDR1-3 set forth in SEQ ID NOs: 6, 17 and 29, respectively, and the light chain variable region having the amino acid sequences of LCDR1-3 set forth in SEQ ID NOs: 40, 47 and 54, respectively; the 4-1BB-targeting domain comprises the amino acid sequences of HCDR1-3 set forth in SEQ ID NOs: 7, 18 and 30, respectively;

v) in the bispecific antibody I, the B7-H4-targeting domain comprises the VH_A having the amino acid sequences of HCDR1-3 set forth in SEQ ID NOs: 100, 104 and 102, respectively, and the VH_B having the amino acid sequences set forth in SEQ ID NOs: 97, 103 and 99, respectively, and the CD3-targeting domain comprises the heavy chain variable region having the amino acid sequences of HCDR1-3 set forth in SEQ ID NOs: 8, 19 and 31, respectively, and the light chain variable region having the amino acid sequences of LCDR1-3 set forth in SEQ ID NOs: 41, 48 and 55, respectively; in the bispecific antibody II, the B7-H4-targeting domain comprises the heavy chain variable region having the amino acid sequences of HCDR1-3 set forth in SEQ ID NOs: 9, 20 and 32, respectively, and the light chain variable region having the amino acid sequences of LCDR1-3 set forth in SEQ ID NOs: 42, 49 and 56, respectively; the 4-1BB-targeting domain comprises the amino acid sequences of HCDR1-3 set forth in SEQ ID NOs: 7, 22 and 30, respectively; and

vi) in the bispecific antibody I, the B7-H4-targeting domain comprises the VH_A having the amino acid sequences of HCDR1-3 set forth in SEQ ID NOs: 100, 104 and 102, respectively, and the VH_B having the amino acid sequences set forth in SEQ ID NOs: 97, 103 and 99, respectively, and the CD3-targeting domain comprises the heavy chain variable region having the amino acid sequences of HCDR1-3 set forth in SEQ ID NOs: 8, 19 and 31, respectively, and the light chain variable region having the amino acid sequences of LCDR1-3 set forth in SEQ ID NOs: 41, 48 and 55, respectively; in the bispecific antibody II, the Her2-targeting domain comprises the heavy chain variable region having the amino acid sequences of HCDR1-3 set forth in SEQ ID NOs: 6, 17 and 29, respectively, and the light chain variable region having the amino acid sequences of LCDR1-3 set forth in SEQ ID NOs: 40, 47 and 54, respectively; the 4-1BB-targeting domain comprises the amino acid sequences of HCDR1-3 set forth in SEQ ID NOs: 7, 18 and 30, respectively.

4. The BsAb combination according to any one of claims 1 to 3, wherein in the bispecific antibody I, the B7-H4-targeting domain comprises a heavy chain variable region having an amino acid sequence set forth in SEQ ID NO: 66 and a light chain variable region having an amino acid sequence set forth in SEQ ID NO: 72; the CD3-targeting domain comprises a heavy chain variable region having an amino acid sequence set forth in SEQ ID NO: 67 and a light chain variable region having an amino acid sequence set forth in SEQ ID NO: 70; or, the B7-H4-targeting domain comprises a heavy chain variable region having an amino acid sequence set forth in SEQ ID NO: 66 and a light chain variable region having an amino acid sequence set forth in SEQ ID NO: 72; the CD3-targeting domain comprises a heavy chain variable region having an amino acid sequence set forth in SEQ ID NO: 64 and a light chain variable region having an amino acid sequence set forth in SEQ ID NO: 70; or, the B7-H4-targeting domain has an amino acid sequence set forth in SEQ ID NO: 122, and the CD3-targeting domain comprises a heavy chain variable region having an amino acid sequence set forth in SEQ ID NO: 67 and a light chain variable region having an amino acid sequence set forth in SEQ ID NO: 70; or, the B7-H4-targeting domain has an amino acid sequence set forth in SEQ ID NO: 123, and the CD3-targeting domain comprises a heavy chain variable region having an amino acid sequence set forth in SEQ ID NO: 64 and a light chain variable region having an amino acid sequence set forth in SEQ ID NO: 70; and/or, the bispecific antibody II is selected from the group consisting of:

a) the bispecific antibody II comprises the B7-H4-targeting domain and the 4-1BB-targeting domain; wherein,

in the B7-H4-targeting domain, the heavy chain variable region has an amino acid sequence set forth in SEQ ID NO: 65, and the light chain variable region has an amino acid sequence set forth in SEQ ID NO: 71; in the 4-1BB-targeting domain, the VH has an amino acid sequence set forth in SEQ ID NO: 68; or, in the B7-H4-targeting domain, the heavy chain variable region has an amino acid sequence set forth in SEQ ID NO: 66, and the light chain variable region has an amino acid sequence set forth in SEQ ID NO: 72; in the 4-1BB-targeting domain, the VH has an amino acid sequence set forth in SEQ ID NO: 63; and

b) the bispecific antibody II comprises the Her2-targeting domain and the 4-1BB-targeting domain; wherein in the Her2-targeting domain, the heavy chain variable region has an amino acid sequence set forth in SEQ ID NO: 62, and the light chain variable region has an amino acid sequence set forth in SEQ ID NO: 69; in the 4-1BB-targeting domain, the VH has an amino acid sequence set forth in SEQ ID NO: 63;

preferably, the BsAb combination is selected from:

i) in the bispecific antibody I, the B7-H4-targeting domain comprises the heavy chain variable region having the amino acid sequence set forth in SEQ ID NO: 66 and the light chain variable region having the amino acid sequence set forth in SEQ ID NO: 72; the CD3-targeting domain comprises the heavy chain variable region having the amino acid sequence set forth in SEQ ID NO: 67 and the light chain variable region having the amino acid sequence set forth in SEQ ID NO: 70; in the bispecific antibody II, the B7-H4-targeting domain comprises the heavy chain variable region having the amino acid sequence set forth in SEQ ID NO: 65, and the light chain variable region having the amino acid sequence set forth in SEQ ID NO: 71; the 4-1BB-targeting domain comprises the VH having the amino acid sequence set forth in SEQ ID NO: 68;

ii) in the bispecific antibody I, the B7-H4-targeting domain comprises the heavy chain variable region having the amino acid sequence set forth in SEQ ID NO: 66 and the light chain variable region having the amino acid sequence set forth in SEQ ID NO: 72; the CD3-targeting domain comprises the heavy chain variable region having the amino acid sequence set forth in SEQ ID NO: 67 and the light chain variable region having the amino acid sequence set forth in SEQ ID NO: 70; in the bispecific antibody II, the Her2-targeting domain comprises the heavy chain variable region having the amino acid sequence set forth in SEQ ID NO: 62, and the light chain variable region having the amino acid sequence set forth in SEQ ID NO: 69; the 4-1BB-targeting domain comprises the VH having the amino acid sequence set forth in SEQ ID NO: 63;

iii) in the bispecific antibody I, the B7-H4-targeting domain comprises the amino acid sequence set forth in SEQ ID NO: 122; the CD3-targeting domain comprises the heavy chain variable region having the amino acid sequence set forth in SEQ ID NO: 67, and the light chain variable region having the amino acid sequence set forth in SEQ ID NO: 70; in the bispecific antibody II, the B7-H4-targeting domain comprises the heavy chain variable region having the amino acid sequence set forth in SEQ ID NO: 65, and the light chain variable region having the amino acid sequence set forth in SEQ ID NO: 71; the 4-1BB-targeting domain comprises the VH having the amino acid sequence set forth in SEQ ID NO: 68;

iv) in the bispecific antibody I, the B7-H4-targeting domain comprises the amino acid sequence set forth in SEQ ID NO: 122; the CD3-targeting domain comprises the heavy chain variable region having the amino acid sequence set forth in SEQ ID NO: 67, and the light chain variable region having the amino acid sequence set forth in SEQ ID NO: 70; in the bispecific antibody II, the Her2-targeting domain comprises the heavy chain variable region having the amino acid sequence set forth in SEQ ID NO: 62, and the light chain variable region having the amino acid sequence set forth in SEQ ID NO: 69; the 4-1BB-targeting domain comprises the VH having the amino acid sequence set forth in SEQ ID NO: 63;

v) in the bispecific antibody I, the B7-H4-targeting domain comprises the amino acid sequence set forth in SEQ ID NO: 123, and the CD3-targeting domain comprises the heavy chain variable region having the amino acid sequence set forth in SEQ ID NO: 67 and the light chain variable region having the amino acid sequence set forth in SEQ ID NO: 70; in the bispecific antibody II, the B7-H4-targeting domain comprises the heavy chain variable region having the amino acid sequence set forth in SEQ ID NO: 65, and the light chain variable region having the amino acid sequence set forth in SEQ ID NO: 71; the 4-1BB-targeting domain comprises the VH having the amino acid sequence set forth in SEQ ID NO: 68; and

vi) in the bispecific antibody I, the B7-H4-targeting domain comprises the amino acid sequence set forth in SEQ ID NO: 123, and the CD3-targeting domain comprises the heavy chain variable region having the amino acid sequence set forth in SEQ ID NO: 67 and the light chain variable region having the amino acid sequence set forth in SEQ ID NO: 70; in the bispecific antibody II, the Her2-targeting domain comprises the heavy chain variable region having the amino acid sequence set forth in SEQ ID NO: 62, and the light chain variable region having the amino acid sequence set forth in SEQ ID NO: 69; the 4-1BB-targeting domain comprises the VH having the amino acid sequence set forth in SEQ ID NO: 63.

5. The BsAb combination according to any one of claims 1 to 4, wherein in the bispecific antibody I, the B7-H4-targeting domain has amino acid sequences set forth in SEQ ID NOs: 92, 115 and 116, and the CD3-targeting domain comprises a heavy chain and a light chain having amino acid sequences set forth in SEQ ID NO: 78 and SEQ ID NO: 81, respectively; or, the B7-H4-targeting domain has amino acid sequences set forth in SEQ ID NOs: 92, 115 and 116, and the CD3-targeting domain comprises a heavy chain and a light chain having amino acid sequences set forth in SEQ ID NO: 75 and SEQ ID NO: 81, respectively; and/or,

in the bispecific antibody II, the B7-H4-targeting domain comprises a heavy chain and a light chain having amino acid sequences set forth in SEQ ID NO: 76 and SEQ ID NO: 82, or SEQ ID NO: 77 and SEQ ID NO: 83, respectively; the Her2-targeting domain comprises a heavy chain and a light chain having amino acid sequences set forth in SEQ ID NO: 73 and SEQ ID NO: 80, respectively; and/or, the 4-1BB-targeting domain comprises a heavy chain having an amino acid sequence set forth in SEQ ID NO: 74 or 79;
preferably, the BsAb combination is selected from:

i) in the bispecific antibody I, the B7-H4-targeting domain comprises the amino acid sequence set forth in SEQ ID NO: 92, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain comprises the amino acid sequence set forth in SEQ ID NO: 79; and the N-terminus of the 4-1BB-targeting domain is directly linked to the C-terminus of the heavy chain of the B7-H4-targeting domain;

ii) in the bispecific antibody I, the B7-H4-targeting domain comprises the amino acid sequence set forth in SEQ ID NO: 92, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain comprises the amino acid sequence set forth in SEQ ID NO: 79; and the N-terminus of the 4-1BB-targeting domain is linked to the C-terminus of the heavy chain of the B7-H4-targeting domain via the linker peptide as set forth in SEQ ID NO: 95;

iii) in the bispecific antibody I, the B7-H4-targeting domain comprises the amino acid sequence set forth in SEQ ID NO: 92, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 73 and SEQ ID NO: 80, respectively, and the 4-1BB-targeting domain comprises the amino acid sequence set forth in SEQ ID NO: 74;

iv) in the bispecific antibody I, the B7-H4-targeting domain comprises the amino acid sequence set forth in SEQ ID NO: 115, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain comprises the amino acid sequence set forth in SEQ ID NO: 79; and the N-terminus of the 4-1BB-targeting domain is directly linked to the C-terminus of the heavy chain of the B7-H4-targeting domain;

v) in the bispecific antibody I, the B7-H4-targeting domain comprises the amino acid sequence set forth in SEQ ID NO: 115, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain comprises the amino acid sequence set forth in SEQ ID NO: 79; and the N-terminus of the 4-1BB-targeting domain is linked to the C-terminus of the heavy chain of the B7-H4-targeting domain via the linker peptide as set forth in SEQ ID NO: 95;

vi) in the bispecific antibody I, the B7-H4-targeting domain comprises the amino acid sequence set forth in SEQ ID NO: 115, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 73 and SEQ ID NO: 80, respectively, and the 4-1BB-targeting domain comprises the amino acid sequence set forth in SEQ ID NO: 74;

vii) in the bispecific antibody I, the B7-H4-targeting domain comprises the amino acid sequence set forth in SEQ ID NO: 116, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain comprises the amino acid sequence set forth in SEQ ID NO: 79; and the N-terminus of the 4-1BB-targeting domain is directly linked to the C-terminus of the heavy chain of the B7-H4-targeting domain;

viii) in the bispecific antibody I, the B7-H4-targeting domain comprises the amino acid sequence set forth in SEQ ID NO: 116, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 76 and SEQ ID NO: 82, respectively, and the 4-1BB-targeting domain comprises the amino acid sequence set forth in SEQ ID NO: 79; and the N-terminus of the 4-1BB-targeting domain is linked to the C-terminus of the heavy chain of the B7-H4-targeting domain via the linker peptide as set forth in SEQ ID NO: 95; and

ix) in the bispecific antibody I, the B7-H4-targeting domain comprises the amino acid sequence set forth in SEQ ID NO: 116, and the CD3-targeting domain comprises the heavy chain and the light chain having the amino acid sequences set forth in SEQ ID NO: 78 and SEQ ID NO: 81, respectively; in the bispecific antibody II, the B7-H4-targeting domain comprises the heavy chain and the light chain having the amino acid sequences set forth in SEQ ID NO: 73 and SEQ ID NO: 80, respectively, and the 4-1BB-targeting domain comprises the amino acid sequence set forth in SEQ ID NO: 74.

6.  A BsAb combination, comprising a bispecific antibody I and a bispecific antibody II, or a bispecific antibody I and urelumab, wherein the bispecific antibody I comprises 3 polypeptide chains: polypeptide chain-1, polypeptide chain-2, and polypeptide chain-3, the amino acid sequences of which are set forth in SEQ ID NO: 81, SEQ ID NO: 88, and SEQ ID NO: 87; or SEQ ID NO: 81, SEQ ID NO: 86, and SEQ ID NO: 87; or SEQ ID NO: 113, SEQ ID NO: 114, and SEQ ID NO: 115; or SEQ ID NO: 113, SEQ ID NO: 114, and SEQ ID NO: 116, respectively;

and/or, the bispecific antibody II comprises 2 polypeptide chains: a short chain and a long chain, wherein the amino acid sequences of the short chain and the long chain are set forth in SEQ ID NO: 82 and SEQ ID NO: 85; or SEQ ID NO: 82 and SEQ ID NO: 89; or SEQ ID NO: 82 and SEQ ID NO: 90; or SEQ ID NO: 83 and SEQ ID NO: 91; or SEQ ID NO: 80 and SEQ ID NO: 84, respectively;

preferably, the BsAb combination is selected from:

i) the bispecific antibody I comprises 3 polypeptide chains having the amino acid sequences set forth in SEQ ID NO: 81, SEQ ID NO: 88 and SEQ ID NO: 87, respectively; the bispecific antibody II comprises 2 polypeptide chains having the amino acid sequences set forth in SEQ ID NO: 82 and SEQ ID NO: 89, respectively;

ii) the bispecific antibody I comprises 3 polypeptide chains having the amino acid sequences set forth in SEQ ID NO: 81, SEQ ID NO: 88 and SEQ ID NO: 87, respectively; the bispecific antibody II comprises 2 polypeptide chains having the amino acid sequences set forth in SEQ ID NO: 82 and SEQ ID NO: 85, respectively;

iii) the bispecific antibody I comprises 3 polypeptide chains having the amino acid sequences set forth in SEQ ID NO: 81, SEQ ID NO: 88 and SEQ ID NO: 87, respectively; the bispecific antibody II comprises 2 polypeptide chains having the amino acid sequences set forth in SEQ ID NO: 80 and SEQ ID NO: 84, respectively;

iv) the bispecific antibody I comprises 3 polypeptide chains having the amino acid sequences set forth in SEQ ID NO: 113, SEQ ID NO: 114 and SEQ ID NO: 115, respectively; the bispecific antibody II comprises 2 polypeptide chains having the amino acid sequences set forth in SEQ ID NO: 82 and SEQ ID NO: 89, respectively;

v) the bispecific antibody I comprises 3 polypeptide chains having the amino acid sequences set forth in SEQ ID NO: 113, SEQ ID NO: 114 and SEQ ID NO: 115, respectively; the bispecific antibody II comprises 2 polypeptide chains having the amino acid sequences set forth in SEQ ID NO: 82 and SEQ ID NO: 85, respectively;

vi) the bispecific antibody I comprises 3 polypeptide chains having the amino acid sequences set forth in SEQ ID NO: 113, SEQ ID NO: 114 and SEQ ID NO: 115, respectively; the bispecific antibody II comprises 2 polypeptide chains having the amino acid sequences set forth in SEQ ID NO: 80 and SEQ ID NO: 84, respectively;

vii) the bispecific antibody I comprises 3 polypeptide chains having the amino acid sequences set forth in SEQ ID NO: 113, SEQ ID NO: 114 and SEQ ID NO: 116, respectively; the bispecific antibody II comprises 2 polypeptide chains having the amino acid sequences set forth in SEQ ID NO: 82 and SEQ ID NO: 89, respectively;

viii) the bispecific antibody I comprises 3 polypeptide chains having the amino acid sequences set forth in SEQ ID NO: 113, SEQ ID NO: 114 and SEQ ID NO: 116, respectively; the bispecific antibody II comprises 2 polypeptide chains having the amino acid sequences set forth in SEQ ID NO: 82 and SEQ ID NO: 85, respectively; and

ix) the bispecific antibody I comprises 3 polypeptide chains having the amino acid sequences set forth in SEQ ID NO: 113, SEQ ID NO: 114 and SEQ ID NO: 116, respectively; the bispecific antibody II comprises 2 polypeptide chains having the amino acid sequences set forth in SEQ ID NO: 80 and SEQ ID NO: 84, respectively.

7. An isolated nucleic acid, encoding the bispecific antibody I and the bispecific antibody II of the BsAb combination according to any one of claims 1 to 6.

8. A recombinant expression vector, comprising the isolated nucleic acid according to claim 7.

9. A transformant, comprising the isolated nucleic acid according to claim 7 or the recombinant expression vector according to claim 8; preferably, the host of the transformant is a prokaryotic cell, preferably an escherichia coli cell, or a eukaryotic cell, preferably a yeast cell or a mammalian cell.

10. A pharmaceutical composition, comprising the BsAb combination according to any one of claims 1 to 6, and preferably, further comprising a third therapeutic agent such as an immune checkpoint antibody and/or a chemotherapeutic agent.

11. A kit, comprising the BsAb combination according to any one of claims 1 to 6, the isolated nucleic acid according to claim 7, the recombinant expression vector according to claim 8, the transformant according to claim 9, or the pharmaceutical composition according to claim 10, and optionally, instructions.

12. Use of the BsAb combination according to any one of claims 1 to 6, the isolated nucleic acid according to claim 7, the recombinant expression vector according to claim 8, the transformant according to claim 9, or the pharmaceutical composition according to claim 10 in the preparation of a medicament for treating cancer; wherein preferably, the cancer is a hematologic tumor or a solid tumor, and the solid tumor is preferably breast cancer, ovarian cancer, or endometrial cancer.

13. A kit of parts, comprising a kit I and a kit II, wherein the kit I comprises the bispecific antibody I of the BsAb combination according to any one of claims 1 to 6, and the kit II comprises the bispecific antibody II of the BsAb combination according to any one of claims 1 to 6; or,
the kit I comprises the bispecific antibody I and the bispecific antibody II of the BsAb combination according to any one of claims 1 to 6; the kit II comprises a third therapeutic agent such as an immune checkpoint antibody and/or a chemotherapeutic agent.

14. An administration device, comprising: (1) an infusion module for administering to a subject in need thereof the pharmaceutical composition according to claim 10, and (2) optionally a pharmacodynamic monitoring module.

15. A method for treating cancer, comprising administered to a subject in need thereof the BsAb combination according to any one of claims 1-6, the pharmaceutical composition according to claim 10, the kit of parts according to claim 13, or the administration device according to claim 14; preferably, wherein the cancer is a hematologic tumor or a solid tumor, and the solid tumor is preferably breast cancer, ovarian cancer, or endometrial cancer.

16. The method according to claim 15, wherein the bispecific antibody I and the bispecific antibody II of the BsAb combination are administered simultaneously or sequentially.

A

**B7H4 x CD3**   Fab-Fc-scFv(VL-VH)

Polypeptide chain-1    N' — | VL_A | CL | — C'

Polypeptide chain-2    N' — | VH_A | CH1 | h | CH2 | CH3 | — C'

Polypeptide chain-3    N' — | VL_B | L | VH_B | h | CH2 | CH3 | — C'

Linker peptid

Hinge region (or linker peptide)

B

**B7H4 x 4-1BB**
**HER2 x 4-1BB**   IgG_HC-VH

Polypeptide chain-1    N' — | VL_A | CL | — C'

Polypeptide chain-2    N' — | VH_A | CH1 | h | CH2 | CH3 | L | VH_B | — C'

Hinge region

Linker peptide

FIG. 1

A

**Binding to CHOK1/hu B7H4**

B

**Binding to CHOK1/cyno B7H4**

C

**Binding to CHOK1/ m B7H4**

D

| Antibody | CHO-h B7H4 | | CHO-cyno B7H4 | | CHO-m B7H4 | |
|---|---|---|---|---|---|---|
| | Maximum MFI | EC50 (nM) | Maximum MFI | EC50 (nM) | Maximum MFI | EC50 (nM) |
| PR003733 | 2281082 | 7.695 | 1771233 | 5.834 | ~3674989 | ~250 |
| PR003899 | 2158028 | 6.06 | 1698851 | 4.958 | ~2205644 | ~62.52 |

FIG. 2

**A**

Binding to SK-BR-3 tumor cells

MFI / Antibody concentration (nM)

PR003733
PR003899
IgG1

**B**

Binding to MDA-MB-468 tumor cells

MFI / Antibody concentration (nM)

PR003733
PR003899
IgG1

**C**

Binding to T cells

MFI / Antibody concentration (nM)

PR003733
PR003899
IgG1

**D**

| Antibody | SK-BR-3 | | MDA-MB-468 | | T cell | |
|---|---|---|---|---|---|---|
| | Maximum MFI | EC50 (nM) | Maximum MFI | EC50 (nM) | Maximum MFI | EC50 (nM) |
| PR003733 | ~224143 | ~23.2 | ~368121 | ~71.15 | ~130263 | ~59.3 |
| PR003899 | ~200052 | ~17.49 | ~346061 | ~51.2 | ~ 5714 | ~ 201.0 |

FIG. 3

EP 4 357 362 A1

A

**Killing MDA-MB-468**

B

**Release level of IFN-γ**

| Antibody | Maximum killing rate % | EC50 (nM) |
|---|---|---|
| PR003733 | 94.9 | 0.005428 |
| PR003899 | 94.82 | 0.05861 |

| Antibody | Maximum IFN-g release value (pg/mL) | EC50 (nM) |
|---|---|---|
| PR003733 | 917.8 | 0.06551 |
| PR003899 | 754.5 | 0.3576 |

FIG. 4

A

**Killing HCC-1954**

| Antibody | Maximum killing rate % | EC50 (nM) |
|---|---|---|
| PR003733 | 95.05 | 0.09094 |
| PR003899 | 80.5 | 0.8765 |

B

**Release level of IFN-γ**

| Antibody | Maximum IFN-g release value (pg/mL) | EC50 (nM) |
|---|---|---|
| PR003733 | 643.4 | 0.174 |
| PR003899 | 339.4 | 0.5888 |

FIG. 5

A  Killing BT-474

| Antibody | Maximum killing rate % | EC50 (nM) |
|---|---|---|
| PR003733 | 89.81 | 0.04281 |
| PR003899 | 69.1 | ~ 0.3770 |

B  Release level of IFN-γ

| Antibody | Maximum IFN-g release value (pg/mL) | EC50 (nM) |
|---|---|---|
| PR003733 | 932.9 | 0.1145 |
| PR003899 | 549.7 | 1.155 |

FIG. 6

**Binding to CHO-K1/h 4-1BB cells**

PR004281
PR004282
Urelumab
IgG1

MFI (y-axis): 0, 100000, 200000, 300000, 400000
Antibody concentration (nM): 0.0001, 0.001, 0.01, 0.1, 1, 10, 100, 1000, 10000

A

**Binding to CHO-K1/cyno 4-1BB cells**

PR004281
PR004282
Urelumab
IgG1

MFI (y-axis): 0, 50000, 100000, 150000, 200000
Antibody concentration (nM): 0.0001, 0.001, 0.01, 0.1, 1, 10, 100, 1000, 10000

B

| Antibody | CHO-K1/h 4-1BB | | CHO-K1/cyno 4-1BB | |
|---|---|---|---|---|
| | Maximum MFI | EC50 (nM) | Maximum MFI | EC50 (nM) |
| PR004281 | 287906 | 2.627 | 287906 | 2.627 |
| PR004282 | 299284 | 2.851 | 299284 | 2.851 |
| Urelumab | 230882 | 1.63 | ~ | ~ |

C

FIG. 7

38

A

**Binding to CHO-K1/h B7H4 cells**

B

**Binding to CHO-K1/cyno B7H4 cells**

C

**Binding to CHO-K1/m B7H4 cells**

D

**Binding to SK-BR-3 tumor cells**

E

| Antibody | CHO-K1/h B7H4 | | CHO-K1/cyno B7H4 | | CHO-K1/m B7H4 | | SK-BR-3 | |
|---|---|---|---|---|---|---|---|---|
| | Maximum MFI | EC50 (nM) | Maximum MFI | EC50 (nM) | Maximum MFI | EC50 (nM) | Maximum MFI | EC50 (nM) |
| PR004281 | 1373931 | 4.037 | 1238578 | 3.972 | 173656 | 3.571 | 23400 | 0.4801 |
| PR004282 | 1249032 | 4.961 | 1202740 | 4.272 | 182405 | 5.192 | 23879 | 0.5444 |

FIG. 8

FIG. 9

EP 4 357 362 A1

**IHC staining of human breast cancer tissue**

A

B

FIG. 10

A
**Killing MDA-MB-468 tumor cells for 24 h**

B
**Killing MDA-MB-468 tumor cells for 48 h**

C
**Killing MDA-MB-468 tumor cells for 72 h**

D
**Killing MDA-MB-468 tumor cells for 96 h**

FIG. 11

FIG. 12

FIG. 13

FIG. 14

FIG. 15

FIG. 16

A

**Binding to CHOK1/hu B7H4**

B

**Binding to CHOK1/cyno B7H4**

C

**Binding to CHOK1/ m B7H4**

| Antibody | CHO-h B7H4 | | CHO-cyno B7H4 | | CHO-m B7H4 | |
|---|---|---|---|---|---|---|
| | Maximum MFI | EC50 (nM) | Maximum MFI | EC50 (nM) | Maximum MFI | EC50 (nM) |
| PR007078 | 1093332 | 1.607 | 1279983 | 1.576 | 546999 | 3.567 |
| PR007168 | 1259615 | 2.074 | 1361031 | 2.194 | 307858 | 165.8 |

FIG. 17

FIG. 18

EP 4 357 362 A1

A

**Killing MDA-MB-468**

| Antibody | Maximum killing rate % | EC50 (nM) |
|---|---|---|
| PR007078 | 105.2 | ~ 5.535e-04 |
| PR007168 | 103.9 | ~ 2.719e-010 |

B

**Release level of IFN-γ**

| Antibody | Maximum IFN-g release value (pg/mL) | EC50 (nM) |
|---|---|---|
| PR007078 | 1862 | 0.03085 |
| PR007168 | 2075 | 0.01257 |

FIG. 19

A

**Killing HCC-1954**

| Antibody | Maximum killing rate % | EC50 (nM) |
|---|---|---|
| PR007078 | 75.97 | 0.05358 |
| PR007168 | 82.79 | 0.01132 |

B

**Release level of IFN-γ**

| Antibody | Maximum IFN-g release value (pg/mL) | EC50 (nM) |
|---|---|---|
| PR007078 | 500.7 | 1.536 |
| PR007168 | 473.5 | 0.05636 |

FIG. 20

EP 4 357 362 A1

FIG. 21

FIG. 22

FIG. 23

FIG. 24

## INTERNATIONAL SEARCH REPORT

| International application No. |
| --- |
| **PCT/CN2022/099607** |

**A.    CLASSIFICATION OF SUBJECT MATTER**

C07K 16/28(2006.01)i;  A61K 39/395(2006.01)i;  A61P 35/00(2006.01)i;  C12N 5/0783(2010.01)i;  C12N 15/09(2006.01)i

According to International Patent Classification (IPC) or to both national classification and IPC

**B.    FIELDS SEARCHED**

Minimum documentation searched (classification system followed by classification symbols)

C07K,  A61K,  A61P,  C12N

Documentation searched other than minimum documentation to the extent that such documents are included in the fields searched

Electronic data base consulted during the international search (name of data base and, where practicable, search terms used)

CNABS, VEN, CNKI, CNTXT, USTXT, EPTXT, PUBMED, ISI WEB, NCBI, EMBL-EBI, STNext: 和铂医药（上海）有限责任公司, 吴晓东, 钟琛, 杜芳芳, 贾鸽子, 王永强, 石磊, 何云, 戎一平, 双特异性抗体, CD3, 4-1BB, B7-H4, Her2, 组合, 联用, T细胞, 免疫检查点, bispecific antibody, composition, combination, T cell, checkpoint, SEQ ID NOs: 6-10, 17-21, 29-33, 40-43, 47-49, 54-57, 62-76, 81, 87-88, 97-116

**C.    DOCUMENTS CONSIDERED TO BE RELEVANT**

| Category* | Citation of document, with indication, where appropriate, of the relevant passages | Relevant to claim No. |
| --- | --- | --- |
| X | CLAUS, C. et al. "Tumor-targeted 4-1BB agonists for combination with T cell bispecific antibodies as off-the-shelf therapy" *Science Translational Medicine*, Vol. 11, 30 June 2019 (2019-06-30), abstract, results part, fig. S5E | 1, 2, 7-16 |
| Y | CLAUS, C. et al. "Tumor-targeted 4-1BB agonists for combination with T cell bispecific antibodies as off-the-shelf therapy" *Science Translational Medicine*, Vol. 11, 30 June 2019 (2019-06-30), abstract, results part, fig. S5E | 1, 2, 7-16 |
| Y | CN 110799540 A (SYSTIMMUNE INC. et al.) 14 February 2020 (2020-02-14) claims 1-26 | 1, 2, 7-16 |
| Y | WO 2021063330 A1 (HARBOUR BIOMED (SUZHOU) CO., LTD.) 08 April 2021 (2021-04-08) claims 1-15 | 1-16 |
| PY | WO 2022002036 A1 (HARBOUR BIOMED (SHANGHAI) CO., LTD.) 06 January 2022 (2022-01-06) claims 1-25 | 1-16 |

☑ Further documents are listed in the continuation of Box C.          ☑ See patent family annex.

| | |
| --- | --- |
| *    Special categories of cited documents: <br> "A"  document defining the general state of the art which is not considered to be of particular relevance <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search | Date of mailing of the international search report |
| --- | --- |
| **05 September 2022** | **16 September 2022** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/099607** |

| C. | DOCUMENTS CONSIDERED TO BE RELEVANT | |
|---|---|---|
| Category* | Citation of document, with indication, where appropriate, of the relevant passages | Relevant to claim No. |
| PY | WO 2022002038 A1 (HARBOUR BIOMED (SHANGHAI) CO., LTD.) 06 January 2022 (2022-01-06)<br>        claims 1-17 | 1-16 |
| PY | WO 2022002033 A1 (HARBOUR BIOMED (SHANGHAI) CO., LTD.) 06 January 2022 (2022-01-06)<br>        claims 1-19 | 1-16 |
| A | RADER, C. "Bispecific antibodies in cancer immunotherapy."<br>*CURRENT OPINION IN BIOTECHNOLOGY*, Vol. 65, 13 December 2019 (2019-12-13),<br>        pp. 9-16 | 1-16 |
| A | CHIU, D. et al. "A PSMA-Targeting CD3 Bispecific Antibody Induces Antitumor Responses that Are Enhanced by 4-1BB Costimulation."<br>*CANCER IMMUNOLOGY RESEARCH*, Vol. 8, No. 5, 31 May 2020 (2020-05-31),<br>        pp. 596-608 | 1-16 |

Form PCT/ISA/210 (second sheet) (January 2015)

**INTERNATIONAL SEARCH REPORT**

International application No.

**PCT/CN2022/099607**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.

**PCT/CN2022/099607**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **15-16**
because they relate to subject matter not required to be searched by this Authority, namely:

    [1]   The subject matter of claims 15-16 relates to a disease treatment method, and therefore, said claims do not comply with PCT Rule 39.1(iv). The present report is provided on the basis that the subject matter is amended to be a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2022/099607**

| Patent document cited in search report | | | Publication date (day/month/year) | Patent family member(s) | | | Publication date (day/month/year) |
|---|---|---|---|---|---|---|---|
| CN | 110799540 | A | 14 February 2020 | US | 2020157224 | A1 | 21 May 2020 |
| | | | | IL | 271260 | A | 30 January 2020 |
| | | | | WO | 2019005640 | A2 | 03 January 2019 |
| | | | | KR | 20200092302 | A | 03 August 2020 |
| | | | | EP | 3645048 | A2 | 06 May 2020 |
| | | | | RU | 2020102663 | A | 27 July 2021 |
| | | | | JP | 2020530306 | A | 22 October 2020 |
| | | | | SG | 11201912865V | A | 30 January 2020 |
| | | | | AU | 2018295119 | A1 | 06 February 2020 |
| | | | | CA | 3068049 | A1 | 03 January 2019 |
| WO | 2021063330 | A1 | 08 April 2021 | KR | 20220071263 | A | 31 May 2022 |
| | | | | TW | 202126694 | A | 16 July 2021 |
| | | | | EP | 4039707 | A1 | 10 August 2022 |
| | | | | AU | 2020359928 | A1 | 21 April 2022 |
| | | | | CA | 3152438 | A1 | 08 April 2021 |
| | | | | CN | 113015749 | A | 22 June 2021 |
| WO | 2022002036 | A1 | 06 January 2022 | TW | 202202529 | A | 16 January 2022 |
| WO | 2022002038 | A1 | 06 January 2022 | TW | 202202524 | A | 16 January 2022 |
| WO | 2022002033 | A1 | 06 January 2022 | TW | 202202530 | A | 16 January 2022 |

Form PCT/ISA/210 (patent family annex) (January 2015)

58

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110678326X
- CN 2022106340425
- WO 2009080251 A
- WO 2009080252 A
- WO 2021063330 A

**Non-patent literature cited in the description**

- **BEJNJAMIN ; STEIN.** *Ther Adv Hematol,* 2016, vol. 7 (3), 142-146
- Bispecific antibodies: a mechanistic review of the pipeline. *Nature Review Drug Discovery,* August 2019, vol. 18 (8), 585-608
- *Transl. Med.,* 2019, vol. 11, eaav5989
- **BERKOW et al.** The Merck Manual of Medical Information. Merck & Co. Inc, 2000
- **EBADI.** Desk Reference of Clinical Pharmacology. CRC, 1998
- *J. Biol. Chem,* 1968, vol. 243, 3558
- **KABAT et al.** Sequences of Proteins of Immunological Interest. National Institutes of Health, 1991
- *J Mol Biol,* 1997, vol. 273, 927-948